(19) Europäisches Patentamt
European Patent Office
Office européen des brevets

(11) EP 4 596 553 A1

(12) EUROPEAN PATENT APPLICATION
published in accordance with Art. 153(4) EPC

(43) Date of publication:
**06.08.2025 Bulletin 2025/32**

(21) Application number: **23884945.9**

(22) Date of filing: **31.10.2023**

(51) International Patent Classification (IPC):
***C07D 491/056*** *(2006.01)* ***A61K 31/519*** *(2006.01)*
***A61P 35/00*** *(2006.01)*

(52) Cooperative Patent Classification (CPC):
**A61K 31/519; A61K 31/5377; A61P 1/00; A61P 1/16; A61P 9/10; A61P 11/00; A61P 17/00; A61P 17/06; A61P 17/14; A61P 19/02; A61P 27/02; A61P 29/00; A61P 35/00; A61P 35/02; A61P 37/06;**
(Cont.)

(86) International application number:
**PCT/CN2023/128656**

(87) International publication number:
**WO 2024/094016 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.11.2022 CN 202211354255**

(71) Applicant: **Beijing Scitech-MQ Pharmaceuticals Limited**
**Beijing 101320 (CN)**

(72) Inventors:
- **ZHANG, Qiang**
**Beijing 101320 (CN)**
- **XU, Jun**
**Beijing 101320 (CN)**
- **JI, Lingtao**
**Beijing 101320 (CN)**

(74) Representative: **Maiwald GmbH**
**Elisenhof**
**Elisenstraße 3**
**80335 München (DE)**

# (54) SALT OF DIOXANE QUINOLINE COMPOUND, CRYSTAL FORM THEREOF, PREPARATION METHODS THEREFOR AND USES THEREOF

(57) The present invention provides a salt of a dioxane quinoline compound, a crystal form thereof, preparation methods therefor and uses thereof. Specifically, the present invention relates to N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, a crystal form thereof and preparation methods therefor, and uses in the preparation of a drug as an inhibitor of tyrosine kinases (e.g. VEGFR-2 and c-MET, etc.).

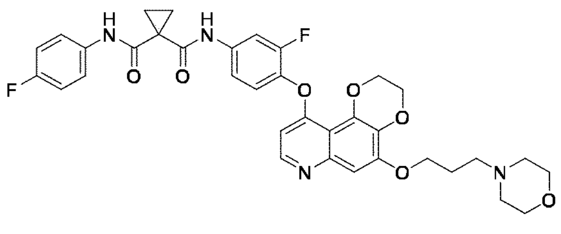

Formula (I)

EP 4 596 553 A1

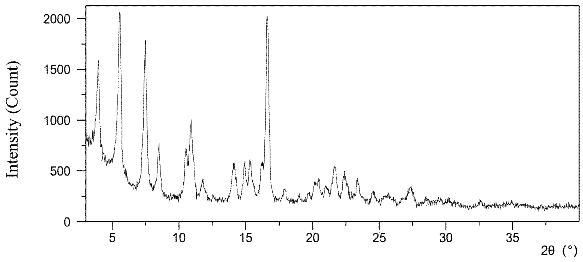


Fig. 1

(52) Cooperative Patent Classification (CPC): (Cont.)
**C07D 491/056**

## Description

### TECHNICAL FIELD

The present disclosure relates to a salt of N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, a crystal form thereof, preparation methods thereof, and uses thereof in the manufacture of a medicament as an inhibitor of tyrosine kinase (e.g., VEGFR-2, c-MET, c-KIT, PDGFRa, RET, AXL, NTRK).

### BACKGROUND

Vascular endothelial growth factor receptor (VEGFR) is a member of the receptor tyrosine kinase family. It binds to its ligand vascular endothelial growth factor (VEGF) to produce a series of biochemical and physiological processes, ultimately promoting the formation of new blood vessels. The formation and permeability of tumor blood vessels are mainly regulated by vascular endothelial growth factor (VEGF), which acts through at least two different receptors (VEGFR-1 and VEGFR-2). According to the research of Jakeman, Kolch, Connolly, etc., VEGF is an important stimulator of normal and pathological angiogenesis and vascular permeability (Jakeman et al., 1993, Endocrinology 133: 848-859; Kolch et al., 1995, Breast Cancer Research and Treatment, 36: 139-155; Connolly et al., 1989, J. Biol. Chem. 264: 20017-20024). Vascular endothelial growth factor induces angiogenic sprouting phenotype by inducing endothelial cell proliferation, protease expression and migration and subsequent formation of capillary cell organization. Therefore, antagonism of VEGF produced by the chelation of VEGF by antibodies may lead to inhibition of tumor growth (Kim et al., 1993, Nature 362: 841-844).

VEGFR-2 can bind to VEGF-A, VEGF-C, VEGF-D, and VEGF-E, since it is mainly distributed in vascular endothelial cells. VEGF stimulates the proliferation of endothelial cells, and increases vascular permeability and the formation of new blood vessels mainly by binding to and activating VEGFR-2. If the activity of VEGFR-2 is blocked, tumor growth and metastasis can be inhibited through direct and indirect pathways, thereby achieving an ideal anti-tumor effect. Therefore, finding small molecule inhibitors with high activity and selectivity for VEGFR-2 has become a very promising tumor treatment strategy.

Hepatocyte growth factor receptor (c-MET) is a type of tyrosine kinase receptor, and its abnormal activation plays an important role in the occurrence and development of various malignant tumors, including lung cancer. Hepatocyte growth factor (HGF) is a specific ligand of c-MET. After binding to HGF, c-MET exerts biological effects through the HGF/c-MET signaling pathway. The HGF/c-MET signaling pathway can induce a series of biological effects such as cell proliferation, dispersion, migration, organ morphogenesis, and angiogenesis. Abnormal activation of c-MET can be manifested as receptor overexpression, gene mutation, amplification, ectopic, and rearrangement, etc. These changes can lead to dysregulation of downstream signaling pathways, such as serine/threonine protein kinase (AKT), extracellular signaling kinase (ERK), phosphatidylinositol-3-hydroxykinase, and retinoblastoma inhibitory protein (Rb) pathways, mediating tumor occurrence, invasion and metastasis, angiogenesis, epithelial-mesenchymal transition and other processes. c-MET plays an important role in cell proliferation, metabolism, tumor generation, metastasis, and angiogenesis, and has become an important target for anti-tumor therapy. Targeted therapy targeting c-MET has shown its importance in the treatment of various malignant tumors including lung cancer. In the treatment process of using anti-tumor drugs, the interaction of multiple signaling pathways will affect the effect of anti-tumor drugs. For example, the interaction between the HFG/c-MET signaling pathway and other pathways affects the therapeutic effect of anti-tumor drugs and produces drug resistance. Therefore, the multi-kinase targets combination therapy has become a new anti-tumor treatment method.

WO2019154133 (Application No. PCT/CN2019/073260, filing date 2019.01.25) discloses a compound that can effectively inhibit VEGFR-2 and c-MET tyrosine kinases. The compound can also effectively inhibit the proliferation of tumor cells such as MHCC97H. The compound structure is shown in formula (I):

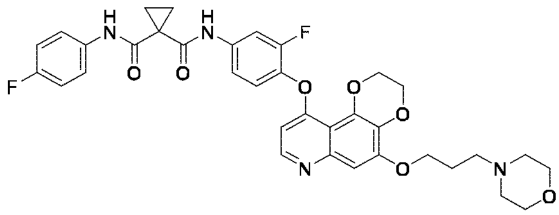

Formula (I)

However, the compound represented by formula (I) exhibits extremely low solubility in water, and unsatisfactory solubility in simulated gastrointestinal fluid, which greatly affects the dissolution of the drug in the gastrointestinal tract and ultimately affects the absorption of the drug in the human body, thereby affecting the efficacy of the drug. Therefore, it is very necessary to improve the physicochemical properties of the compound represented by formula (I) in various aspects (such as solubility, chemical stability, pharmacokinetic properties, and bioavailability, etc.).

SUMMARY

The present disclosure provides pharmaceutically acceptable salts of the compound of formula (I), crystal forms thereof, and methods for preparing the same and their use in the manufacture of a medicament as inhibitors of tyrosine kinases (e.g., VEGFR-2, c-MET, c-KIT, PDGFRa, RET, AXL, NTRK). The salt types and crystal forms of the present disclosure have good physicochemical properties and biochemical activities.

The present disclosure provides a pharmaceutically acceptable salt of a compound represented by formula (I),

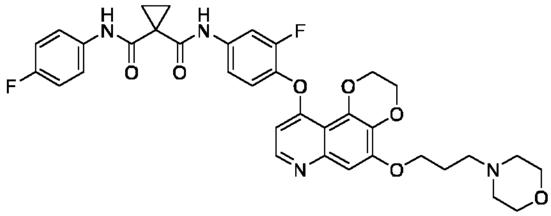

Formula (I)

**The** pharmaceutically acceptable salt is selected from inorganic salts and organic salts, alternatively p-toluenesulfonate, mucate, and phosphate.

Specifically, the pharmaceutically acceptable salt of the present disclosure can be a salt of N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide and p-toluenesulfonic acid in a chemical ratio of 1:1 or 1:2.

The present disclosure provides a method for preparing a pharmaceutically acceptable salt of the compound of formula (I), comprising the step of subjecting N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide to a salt-forming reaction with an inorganic acid or an organic acid, alternatively with p-toluenesulfonic acid, mucic acid or phosphoric acid.

Specifically, the salt-forming reaction is carried out in a solvent, and the solvent is selected from water, alcohol solvents, halogenated hydrocarbon solvents, ketone solvents, ether solvents, nitrile solvents, ester solvents, amide solvents, aliphatic hydrocarbon solvents, alicyclic hydrocarbon solvents, aromatic hydrocarbon solvents, mixed solvents of alcohol solvents and water, mixed solvents of ketone solvents and water, mixed solvents of alcohol solvents and ether solvents, mixed solvents of halogenated hydrocarbon solvents and nitrile solvents, mixed solvents of amide solvents and water, or mixed solvents of nitrile solvents and water; the ketone solvent is alternatively acetone, the alcohol solvent is alternatively methanol, ethanol or isopropanol, the ether solvent is alternatively diethyl ether, methyl tert-butyl ether, tetrahydrofuran, 2-methyltetrahydrofuran or dioxane, the halogenated hydrocarbon solvent is alternatively dichloromethane or chloroform, the nitrile solvent is alternatively acetonitrile, the ester solvent is alternatively ethyl acetate, isopropyl acetate or butyl acetate, the amide solvent is alternatively N,N-dimethylformamide or N,N-dimethylacetamide, the aliphatic hydrocarbon solvent is alternatively n-heptane, the alicyclic hydrocarbon solvent is alternatively cyclohexane, the aromatic hydrocarbon solvent is alternatively toluene, xylene or isopropylbenzene, the mixed solvent of the alcohol solvent and the ether solvent is alternatively a mixed solvent of diethyl ether and methanol, the mixed solvent of the halogenated hydrocarbon solvent and the nitrile solvent is alternatively a mixed solvent of dichloromethane and acetonitrile, the mixed solvent of the alcohol solvent and water is alternatively a mixed solvent of methanol and water or ethanol and water, the mixed solvent of the ketone solvent and water is alternatively a mixed solvent of acetone and water, and the mixed solvent of the amide solvent and water is alternatively a mixed solvent of N,N-dimethylformamide and water.

The present disclosure provides a method for preparing a p-toluenesulfonate salt of the compound of formula (I), comprising the step of forming a salt from N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide and p-toluenesulfonic acid in a chemical ratio of 1:1 or 1:2. The present disclosure further provides a method for preparing a crystal form A-II of p-toluenesulfonate of the compound of formula (I), comprising weighing a certain amount of the compound of formula (I) and p-toluenesulfonic acid in a molar ratio of 1:1, suspending and stirring in a solvent for a period of time, and then drying. More specifically, the solvent may be acetone, dichloromethane or dioxane.

The present disclosure provides a method for preparing a mucate salt of the compound of formula (I), comprising weighing a certain amount of the compound of formula (I) and mucic acid in a molar ratio of 0.5:1, suspending and stirring in a solvent for a period of time, and then vacuum drying. More specifically, the solvent may be acetone.

The present disclosure provides a method for preparing a phosphate salt of the compound of formula (I), comprising weighing a certain amount of the compound of formula (I) and phosphoric acid in a molar ratio of 1:1, suspending and stirring in a solvent for a period of time, and then vacuum drying. More specifically, the solvent may be ethanol / water.

The present disclosure further provides a method for preparing a crystal form of a pharmaceutically acceptable salt of the compound of formula (I), comprising weighing a certain amount of the compound of formula (I) and the inorganic acid or organic acid, alternatively p-toluenesulfonic acid, mucic acid, phosphoric acid, in a certain molar ratio, suspending and stirring in a solvent for a period of time, and then drying. The solvent is as described above. The preparation method of the crystal form of the present disclosure may also include methods such as anti-solvent addition, slow volatilization, slow cooling, suspension stirring at room temperature or high temperature, cyclic heating and cooling, gas-solid permeation, gas-liquid diffusion, high polymer induction, etc.

The present disclosure provides a crystal form A-I of a mucate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 3.95, 5.54, 7.46, 10.87, and 16.58, wherein the error range of 2θ angle of each characteristic peak is ±0.2;

alternatively, there are characteristic peaks at 2θ angles of 3.47, 3.95, 5.54, 7.46, 8.46, 10.48, 10.87, 14.90, 15.28, 16.16, and 16.58, wherein the error range of 2θ angle of each characteristic peak is ±0.2;
yet alternatively, there are characteristic peaks at 2θ angles of 3.47, 3.95, 5.54, 7.46, 8.46, 10.48, 10.87, 11.72, 14.08, 14.90, 15.28, 16.16, 16.58, 17.90, 20.29, 21.65, 22.35, 23.37, 24.53, 25.45, 27.35, and 32.58, wherein the error range of 2θ angle of each characteristic peak is ±0.2.

The present disclosure provides a crystal form A-II of a p-toluenesulfonate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl) cyclopropane-1,1-dicarboxamide, wherein in its X-ray powder diffraction pattern, there are characteristic peaks at 2θ angles of 5.30, 6.54, 7.97, 8.78, 13.01, 15.86, 19.70, 19.94, and 20.62, wherein the error range of 2θ angle of each characteristic peak is ±0.2;

alternatively, there are characteristic peaks at 2θ angles of 5.30, 6.54, 7.97, 8.78, 10.52, 11.63, 11.81, 13.01, 15.86, 17.54, 19.34, 19.70, 19.94, 20.62, 22.50, and 25.95, wherein the error range of 2θ angle of each characteristic peak is ±0.2;
yet alternatively, there are characteristic peaks at 2θ angles of 5.30, 6.54, 7.97, 8.78, 10.52, 11.63, 11.81, 13.01, 13.54, 14.33, 14.52, 15.06, 15.86, 16.46, 17.54, 18.02, 18.25, 18.75, 19.34, 19.70, 19.94, 20.62, 21.06, 21.46, 22.50, 23.67, 24.02, 24.35, 25.12, 25.95, 26.45, 27.20, 27.87, 29.20, 30.15, 30.87, and 31.83, wherein the error range of 2θ angle of each characteristic peak is ±0.2.

The present disclosure provides a crystal form A-IV of a di-p-toluenesulfonate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl) cyclopropane-1,1-dicarboxamide, wherein in its X-ray powder diffraction pattern, there are characteristic peaks at 2θ angles of 6.36, 13.60, and 19.20, wherein the error range of 2θ angle of each characteristic peak is ±0.2;

alternatively, there are characteristic peaks at 2θ angles of 6.36, 9.87, 11.50, 13.60, 16.00, 19.20, 20.26, 20.86, and 21.36, wherein the error range of 2θ angle of each characteristic peak is ±0.2;
yet alternatively, there are characteristic peaks at 2θ angles of 6.36, 9.45, 9.87, 11.50, 12.88, 13.60, 14.40, 16.00, 19.20, 20.26, 20.86, 21.36, 22.59, 23.76, and 26.73, wherein the error range of 2θ angle of each characteristic peak is ±0.2.

The present disclosure provides a crystal form A-V of a phosphate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 5.68, 7.56, and 16.93, wherein the error range of 2θ angle of each characteristic peak is ±0.2;

alternatively, there are characteristic peaks at 2θ angles of 4.03, 5.68, 6.84, 7.56, 9.36, 11.30, 11.94, 16.93, 19.76, and 23.01, wherein the error range of 2θ angle of each characteristic peak is ±0.2;
yet alternatively, there are characteristic peaks at 2θ angles of 4.03, 5.68, 6.84, 7.56, 9.36, 11.30, 11.94, 12.56, 13.69,

16.17, 16.93, 19.76, 22.02, 23.01, 23.98, and 25.19, wherein the error range of 2θ angle of each characteristic peak is ±0.2.

Another aspect of the present application provides a pharmaceutical composition, which comprises a pharmaceutically acceptable salt of the compound of formula (I) described in the present application, alternatively a p-toluenesulfonate, a mucate, a phosphate, or comprises any one of crystal forms of the mucate salt crystal form A-I, p-toluenesulfonate salt crystal form A-II, di-p-toluenesulfonate salt crystal form A-IV or phosphate salt crystal form A-V of the compound of formula (I), and one or more pharmaceutically acceptable carriers or excipients.

The pharmaceutical composition of the present application may also contain one or more additional therapeutic agents.

The present application also relates to use of a pharmaceutically acceptable salt, crystal form or pharmaceutical composition of the compound of formula (I) in the manufacture of a medicament for treating diseases related to tyrosine kinases VEGFR-2, c-MET, c-KIT, PDGFRa, RET, AXL, or NTRK (NTRK includes TRK-A, TRK-B, TRK-C and other TRK family kinases).

The disease is alternatively cancer or an autoimmune disease, in particular ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, or cholangiocarcinoma.

The present disclosure also relates to a method for treating diseases or conditions mediated by kinases such as VEGFR-2, c-MET, c-KIT, PDGFRa, RET, AXL, NTRK, etc., which comprises administering a therapeutically effective amount of the pharmaceutically acceptable salt, crystal form or pharmaceutical composition of the compound of formula (I) described in the present application to a patient in need (human or other mammal, especially human), wherein the diseases or conditions mediated by kinases such as VEGFR-2, c-MET, c-KIT, PDGFRa, RET, AXL, NTRK, etc. include those mentioned above.

## BRIEF DESCRIPTION OF DRAWINGS

The above and other objects, features and other advantages of the present disclosure will be more clearly understood from the following detailed description in conjunction with the accompanying drawings, in which:

Fig. 1 is an XRPD diagram of the mucate salt crystal form A-I of the compound of formula (I);
Fig. 2 is a TGA graph of the mucate salt crystal form A-I of the compound of formula (I);
Fig. 3 is a DSC graph of the mucate salt crystal form A-I of the compound of formula (I);
Fig. 4 is an XRPD diagram of the p-toluenesulfonate salt crystal form A-II of the compound of formula (I);
Fig. 5 is a TGA graph of the p-toluenesulfonate salt crystal form A-II of the compound of formula (I);
Fig. 6 is a DSC graph of the p-toluenesulfonate salt crystal form A-II of the compound of formula (I);
Fig. 7 is an XRPD diagram of the p-toluenesulfonate salt crystal form B-III of the compound of formula (I);
Fig. 8 is a TGA graph of the p-toluenesulfonate salt crystal form B-III of the compound of formula (I);
Fig. 9 is a DSC graph of the p-toluenesulfonate salt crystal form B-III of the compound of formula (I);
Fig. 10 is an XRPD diagram of the di-p-toluenesulfonate salt crystal form A-IV of the compound of formula (I);
Fig. 11 is a TGA graph of the di-p-toluenesulfonate salt crystal form A-IV of the compound of formula (I);
Fig. 12 is a DSC graph of the di-p-toluenesulfonate salt crystal form A-IV of the compound of formula (I);
Fig. 13 is an XRPD diagram of the phosphate salt crystal form A-V of the compound of formula (I);
Fig. 14 is a TGA graph of the phosphate salt crystal form A-V of the compound of formula (I);
Fig. 15 is a DSC graph of the phosphate salt crystal form A-V of the compound of formula (I);
Fig. 16 is a graph showing changes in tumor volume in the administration group of the free-state of the compound of formula (I) and the negative control group;
Fig. 17 is a graph showing changes in tumor volume in the administration group of the mucate salt crystal form A-I of the compound of formula (I) of Example 1 and the negative control group;
Fig. 18 is a graph showing changes in tumor volume in the administration group of the p-toluenesulfonate salt crystal form A-II of the compound of formula (I) of Example 2 and the negative control group;
Fig. 19 is a graph showing changes in tumor volume in the administration group of the phosphate salt crystal form A-V of the compound of formula (I) of Example 5 and the negative control group.

## DETAILED DESCRIPTION

Unless otherwise stated, the following terms used in this application (including the specification and claims) have the definitions given below. In this application, the use of "or" or "and" means "and/or" unless stated otherwise. In addition, the use of the term "comprising" and other forms such as "including", "containing" and "having" is not limiting. The chapter headings used herein are for organizational purposes only and should not be interpreted as limitations on the topics described.

Unless otherwise specified, the term "$C_1$-$C_6$ alkyl" means an alkyl group containing 1 to 6 carbon atoms. Similarly, $C_1$-$C_3$ alkyl means an alkyl group containing 1 to 3 carbon atoms. For example, $C_1$-$C_6$ alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl and 2-methylpentyl, etc.

The term "ether solvent" refers to a chain compound or a cyclic compound containing an ether bond - O- and having 1 to 10 carbon atoms. Specific examples include, but are not limited to, diethyl ether, dioxane, tetrahydrofuran, 2-methyltetrahydrofuran, propylene glycol methyl ether or methyl tert-butyl ether.

The term "alcohol solvent" refers to a group derived by replacing one or more hydrogen atoms on a $C_1$-$C_6$ alkyl group with one or more hydroxyl groups, and specific examples include but are not limited to methanol, ethanol, isopropanol, n-propanol, isopentanol or trifluoroethanol.

The term "ester solvent" refers to a conjugate of a lower organic acid containing 1 to 4 carbon atoms and a lower alcohol containing 1 to 6 carbon atoms, and specific examples include but are not limited to ethyl acetate, isopropyl acetate or butyl acetate.

The term "ketone solvent" refers to a compound in which a carbon group (-CO-) is connected to two hydrocarbon groups. Depending on the hydrocarbon groups in the molecule, ketones can be categorized as aliphatic ketones, alicyclic ketones, aromatic ketones, saturated ketones and unsaturated ketones. Specific examples include but are not limited to: acetone, acetophenone, methyl isobutyl ketone or methyl pyrrolidone.

The term "nitrile solvent" refers to a group derived by replacing one or more hydrogen atoms on a $C_1$-$C_6$ alkyl group with one or more cyano groups, and specific examples include, but are not limited to, acetonitrile or propionitrile.

The term "aliphatic hydrocarbon solvent" refers to chain hydrocarbons, such as saturated aliphatic hydrocarbons, having 1 to 10 carbon atoms, including alkane solvents, and specific examples include but are not limited to: n-butane, n-pentane, n-hexane or n-heptane.

The term "alicyclic hydrocarbon solvent" refers to a hydrocarbon compound having a cyclic carbon skeleton and 1 to 8 ring atoms, and specific examples include but are not limited to cyclopentane or cyclohexane.

The term "amide solvent" refers to a compound containing a carbonylamino group (-CONH-) and having 1 to 10 carbon atoms, and specific examples include, but are not limited to, N,N-dimethylformamide or N,N-dimethylacetamide.

The term "aromatic hydrocarbon solvent" refers to a general term for carbocyclic compounds and their derivatives having a closed cyclic conjugated system in the molecule, where the number of $\pi$ electrons complies with Huckel's rule. Specific examples include, but are not limited to, toluene, isopropylbenzene or xylene.

The term "halogenated hydrocarbon solvent" refers to a group derived by replacing one or more hydrogen atoms on a $C_1$-$C_6$ alkyl group with one or more halogen atoms, wherein the halogen atoms include fluorine, chlorine, bromine, iodine atoms, etc. Specific examples of the "halogenated hydrocarbon solvent" include, but are not limited to, methyl chloride, dichloromethane, chloroform or carbon tetrachloride.

The term "mixed solvent" refers to a solvent formed by mixing one or more different types of organic solvents in a certain proportion, or a solvent formed by mixing an organic solvent and water in a certain proportion; the mixed solvent is alternatively a mixed solvent of one or more alcohol solvents, a mixed solvent of an alcohol solvent and an ether solvent, a mixed solvent of an alcohol solvent and an aliphatic hydrocarbon solvent, a mixed solvent of an ether solvent and an aliphatic hydrocarbon solvent, a mixed solvent of an alcohol solvent and water, a mixed solvent of a ketone solvent and water, a mixed solvent of a halogenated hydrocarbon solvent and a nitrile solvent, a mixed solvent of an amide solvent and water, or a mixed solvent of an ether solvent and water; wherein the alcohol solvent, ether solvent, aliphatic hydrocarbon solvent, halogenated hydrocarbon solvent, amide solvent and nitrile solvent are as defined above.

As used herein, the term "pharmaceutically acceptable salt" refers to organic and inorganic salts of the compounds of the present disclosure which are suitable for use in human and lower animals without undue toxicity, irritation, allergic response, etc., and have reasonable benefit/risk ratio. Pharmaceutically acceptable salts of amines, carboxylic acids, phosphonates, and other types of compounds are well known in the art. The salt can be formed by reacting a compound of the disclosure with a suitable free base or acid, including, but not limited to, inorganic acids such as hydrochloric acid, hydrobromic acid, phosphoric acid, sulfuric acid, perchloric acid or organic acids such as acetic acid, oxalic acid, maleic acid, tartaric acid, citric acid, succinic acid, malonic acid. Or the salts may be obtained by methods well known in the art, such as ion exchange. Other pharmaceutically acceptable salts include adipate, alginate, ascorbate, aspartate, besylate, benzoate, bisulfate, borate, butyrate, camphorate, camphorsulfonate, citrate, digluconate, lauryl sulfate, ethanesulfonate, formate, fumarate, glucoheptonate, glycerol phosphate, glyconate, hemisulfate, hexanoate,

hydroiodide, 2-hydroxyethanesulfonate, lactobionate, lactate, laurate, lauryl sulfate, malate, maleate, mesylate, 2-naphthalenesulfonate, nicotinate, nitrate, oleate, palmitate, pamoate, pectate, persulphate, per-3-phenylpropionate, phosphate, picrate, propionate, stearate, sulfate, thiocyanate, p-toluenesulfonate, undecanoate, and the like. Representative alkali or alkaline earth metal salts include salts of sodium, lithium, potassium, calcium, magnesium, and the like. Other pharmaceutically acceptable salts include suitable non-toxic salts of ammonium, quaternary ammonium, and amine cations formed from halides, hydroxides, carboxylates, sulfates, phosphates, nitrates, lower alkyl sulfonates and aryl sulfonates.

The pharmaceutical composition of the present disclosure comprises a pharmaceutically acceptable salt of the compound of formula (I) described herein or a crystal form thereof, a kinase inhibitor (small molecule, polypeptide, antibody, etc.), an immunosuppressant, an anticancer drug, an antiviral agent, an anti-inflammatory agent, an antifungal agent, an antibiotic or an additional active agent as an anti-angiogenic compound; and any pharmaceutically acceptable carrier, adjuvant or excipient.

The pharmaceutically acceptable salt of the compound of formula (I) of the present disclosure or its crystal form can be used alone or in combination with one or more other agents. When administered in combination, the therapeutic agents can be formulated for simultaneous or sequential administration at different times, or the therapeutic agents can be administered as a single composition. By "combination therapy", it refers to the use of a compound of the present disclosure in combination with another agent, in a manner whereby each agent is co-administered simultaneously or administered sequentially, in either case, for the purpose of achieving the optimal effect of drugs. Co-administration includes dosage form for simultaneous delivery, as well as separate dosage forms for each compound. Thus, administration of the compounds of the disclosure can be combined with other therapies known in the art, for example, radiation therapy or cytostatic agents, cytotoxic agents, other anticancer agents, and the like as used in the treatment of cancer, in order to improve the symptoms of cancer. The administration sequence is not limited in the present disclosure. The compounds of the present disclosure may be administered before, simultaneously with, or after other anticancer or cytotoxic agents.

To prepare the pharmaceutical ingredient of the present disclosure, the pharmaceutically acceptable salt of the compound of formula (I) or its crystal form as the active ingredient can be intimately mixed with a pharmaceutical carrier, which is carried out according to conventional pharmaceutical formulation techniques. The carrier can be used in a wide variety of forms depending on the form of preparation which is designed for different modes of administration (for example, oral or parenteral administration). Suitable pharmaceutically acceptable carriers are well known in the art. A description of some of these pharmaceutically acceptable carriers can be found in the Handbook of Pharmaceutical Excipients, published jointly by the American Pharmaceutical

Association and the Pharmaceutical Society of Great Britain.

The pharmaceutical composition of the present disclosure may have the following forms, for example, those suitable for oral administration, such as tablets, capsules, pills, powders, sustained release forms, solutions or suspensions; those for parenteral injections such as clear solutions, suspensions, emulsion; or those for topical application such as ointments, creams; or as a suppository for rectal administration. The pharmaceutical ingredients may also be presented in unit dosage form for single administration in a precise dosage. The pharmaceutical ingredient will include a conventional pharmaceutical carrier or excipient and a pharmaceutically acceptable salt of a compound or its crystal form prepared according to the present disclosure as an active ingredient, and may also include other medical or pharmaceutical preparations, carriers, adjuvants, and the like.

Therapeutic compounds can also be administered to mammals other than humans. The drug dosage for a mammal will depend on the species of the animal and its disease condition or its disordered condition. The therapeutic compound can be administered to the animal in the form of a capsule, a bolus, a tablet or liquid. The therapeutic compound can also be introduced into the animal by injection or infusion. These drug forms are prepared in a traditional manner complying with standard veterinary practice. As an alternative, the pharmaceutical synthetic drugs can be fed to the animal in a mixture with the animal feed, so that the concentrated feed additive or premix can be prepared by mixing ordinary animal feed.

## DETAILED DESCRIPTION

In order to make the objectives, technical solutions and advantages of the present disclosure clearer, the present disclosure will be further described in detail below in conjunction with specific examples. It should be understood that the specific examples described here are only used to explain the present disclosure and are not intended to limit the present disclosure. If no specific technology or conditions are indicated in examples, the technology or conditions described in the literature in the art or the product specification shall be followed. If manufacturers are not indicated for reagents or instruments used, the reagents or instruments are all conventional products that are commercially available. The term "and/or" as used herein includes any and all combinations of one or more related listed items. Examples are provided below to better illustrate the disclosure, and all temperatures refer to °C unless otherwise noted. The naming of some

compounds in this application is obtained according to the chemdraw naming translation. The present disclosure also provides methods for preparing corresponding pharmaceutically acceptable salts and crystal forms. A variety of methods can be used to prepare the salts and crystal forms described herein, including the following methods, or using other methods known in the chemical field. Those skilled in the art also understand the changes to these methods, and alternative methods include but are not limited to the following methods.

**Instruments used in the assay and their test conditions:**

X-ray powder diffraction (XRPD): The measurement was carried out using an X'Pert3 X-ray powder diffraction analyzer produced by PANalytacal. The specific collection information is shown in Table 1 below.

Table 1. XRPD test parameters

| Parameter | XRPD |
|---|---|
| model | X' Pert3 |
| X-ray | Cu, Kα, Kα1 (Å):1.540598, Kα2 (Å):1.544426 Kα2/Kα1 intensity ratio:0.50 |
| X-ray tube settings | 45 kV, 40 mA |
| Diverging slits | 1/8° |
| Scan Mode | Continuous |
| Scanning range (°2Theta) | 3-40 |
| Scan time per step (s) | 46.7 |
| Scan step size (°2Theta) | 0.0263 |
| Testing time | ~ 5 min |

TGA thermogravimetric analysis: The test was carried out using a TA Q5000/5500 thermogravimetric analyzer with a heating rate of 10°C/min and a temperature range of room temperature to the set endpoint temperature (refer to the corresponding graph for details).

DSC differential scanning calorimetry: The test was carried out using a TA Q200/Q2000/2500 differential scanning calorimeter with a heating rate of 10 °C/min and a temperature range of 25°C to set endpoint temperature (refer to the corresponding graph for details).

Dynamic moisture sorption (DVS): The curves were collected on a DVS Intrinsic of SMS (Surface Measurement Systems). At 25 °C, the humidity range was 0%RH-95 % RH, the step was 10% (the last step was 5%), and the dm/dt was 0.002%/min.

High performance liquid chromatography/ion chromatography (HPLC/IC): HPLC was tested using an Agilent 1260/1100 high performance liquid chromatograph (Agilent Eclipse Plus C18, 100×4.6 mm, 3.5 μm, chromatographic column), and IC was tested using a ThermoFisher ICS-1100 ion chromatograph (IonPac AS18 Analytical Column, 250*4 mm, chromatographic column).

**Biological solvent Preparation**

1. Preparation of simulated gastric fluid (SGF): 0.1 g of NaCl and 0.05 g of Trinaton X-100 were weighed into a 50-mL volumetric flask and completely dissolved in purified water. 67.5 μL of concentrated hydrochloric acid (12 M) was added and the mixture was adjusted to a pH value of 1.8 with 1 M hydrochloric acid or 1 M NaOH solution. Purified water was added to make up to volume.
2. Preparation of Fasted State Simulated Intestinal Fluid (FaSSIF): 0.17 g of anhydrous $NaH_2PO_4$, 0.021 g of NaOH, and 0.31 g of NaCl were weighed into a 50-mL volumetric flask. About 48 mL of purified water was added to dissolve the mixture completely, and the solution was adjusted to a pH value of 6.5 with 1 M hydrochloric acid or 1 M NaOH solution. Purified water was added to make up to volume. 0.11 g of SIF powder was added and dissolved completely.
3. Preparation of Fed State Simulated Intestinal Fluid (FeSSIF): 0.41 mL of glacial acetic acid, 0.20 g of NaOH and 0.59 g of NaCl were weighed into a 50-mL volumetric flask. About 48 mL of purified water was added to dissolve the mixture completely, and the solution was adjusted to a pH value of 5.0 with 1 M hydrochloric acid or 1 M NaOH solution. Purified water was added to make up to volume. 0.56 g of SIF powder was added and dissolved completely.

**Synthesis of free state of formula (I)**

Preparation of N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide

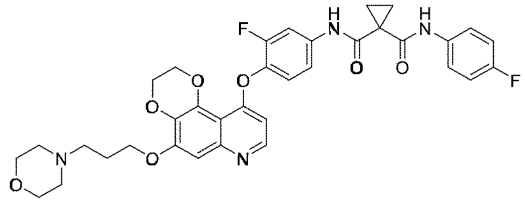

Formula (I)

Its preparation refers to the document of Patent Application No. WO2019154133.

Step 1): 10-chloro-5-methoxy-2,3-dihydro-[1,4]dioxano[2,3-f]quinoline (251 mg, 1 mmol) was dissolved in dichloromethane. 1 M boron tribromide solution in dichloromethane (3 mL, 3 mmol) was added dropwise, and the mixture was stirred until the reaction was completed. The product was concentrated to give 236 mg of a light yellow solid (5-hydroxy-10-chloro-2,3-dihydro-[1,4]dioxano[2,3-f]quinoline) with a yield of 99%. MS: 238$[M+H]^+$.

Step 2): The product obtained in step 1) (236 mg, 1 mmol) was dissolved in N,N-dimethylformamide. 4-(3-chloropropyl)morpholine (163 mg, 1 mmol) and potassium carbonate (414 mg, 3 mmol) were added, and the mixture was heated and stirred until the reaction was completed. Water and ethyl acetate were added to extract the mixture. The organic phase was concentrated and then purified by column chromatography to give 291 mg of an off-white solid (10-chloro-5-(3-morpholinopropoxy)-2,3-dihydro-[1,4]dioxano[2,3-f]-quinoline) with a yield of 80%. $^1$H NMR (400 MHz, DMSO-$d_6$) δ 8.50 (d, J = 4.8 Hz, 1H), 7.37 (d, J = 4.8 Hz, 1H), 7.10 (s, 1H), 4.47-4.30 (m, 4H), 4.17 (t, J = 6.4 Hz, 2H), 3.59 (t, J = 4.6 Hz, 4H), 2.45 (t, J = 7.1 Hz, 2H), 2.39 (d, J = 4.5 Hz, 4H), 1.97-1.95 (m, 2H). MS: 365$[M+H]^+$.

Step 3): Chlorobenzene (5 mL), 10-chloro-5-(3-morpholinopropoxy)-2,3-dihydro-[1,4]dioxano[2,3-f]quinoline (291 mg, 0.8 mmol), 2-fluoro-4-nitrophenol (125 mg, 0.8 mmol) and triethylamine (0.3 mL) were added into a reaction flask and the mixture was reacted under reflux for 15-20 hours. The reaction mixture was cooled and concentrated, and the obtained solid was washed with aqueous potassium carbonate solution to give 370 mg of a yellow solid product (10-(2-fluoro-4-nitrophenoxy)-5-(3-morpholinopropoxy)-2,3-dihydro-[1,4]dioxano[2,3-f]quinoline), with a yield of 96%, MS: 486 $[M+H]^+$.

Step 4): The product obtained in step 3) (370 mg, 0.76 mmol) was added to Pd/C in methanol (10 mL). After hydrogen replacement, the reaction was stirred at room temperature under hydrogen atmosphere for 10 hours. The reaction mixture was filtered and concentrated to give 340 mg of a white solid product (3-fluoro-4-((5-(3-morpholinopropoxy)-2,3-dihydro-[1,4]dioxane[2,3-f]quinolin-10-yl)oxy)aniline), $^1$H NMR (400 MHz, DMSO-$d_6$) δ 8.37 (d, J = 5.3 Hz, 1H), 7.08-6.88 (m, 2H), 6.60-6.49 (m, 1H), 6.48-6.40 (m, 1H), 6.32 (d, J = 5.2 Hz, 1H), 5.44 (s, 2H), 4.37-4.39 (m, 4H), 4.16 (t, J = 6.4 Hz, 2H), 3.59 (t, J = 4.6 Hz, 4H), 2.46 (d, J = 7.0 Hz, 2H), 2.39 (s, 4H), 1.95-1.97 (m, 2H); MS: 456$[M+H]^+$.

Step 5): A solution of 1-(4-fluorophenylcarbamoyl)cyclopropane-1-carboxylic acid (170 mg, 0.76 mmol) in thionyl chloride (5 mL) was heated to reflux, and reacted. After clarification, the reflux reaction was continued to reflux and react for 1 hour. The reaction mixture was cooled, and concentrated to give 180 mg of a yellow solid product (1-(4-fluorophenylcarbamoyl) cyclopropane 1-carbonyl chloride), which was used directly in the next step;

Step 6): To a solution of 3-fluoro-4-((5-(3-morpholinopropoxy)-2,3-dihydro-[1,4]dioxano[2,3-flquinolin-10-yl) oxy)aniline (340 mg, 0.75mmol) in NMP (2 mL) were added a solution of 1-(4-fluorophenylcarbamoyl)cyclopropane 1-carbonyl chloride (180 mg, 0.75 mmol) in dichloromethane (1 mL) and triethylamine (0.2 mL), respectively. The mixture was stirred at room temperature for 5 hours, and quenched with water. The mixture was washed with saturated sodium carbonate, extracted with dichloromethane, dried, concentrated, and purified by column chromatography to give 260 mg of the product (N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide), $^1$H NMR (400 MHz, DMSO-$d_6$) δ 10.32 (s, 1H), 10.00 (s, 1H), 8.41 (d, J = 5.2 Hz, 1H), 7.98 - 7.79 (m, 1H), 7.67 - 7.59 (m, 2H), 7.53 - 7.39 (m, 1H), 7.24 (t, J = 9.0 Hz, 1H), 7.18 - 7.11 (m, 2H), 7.06 (s, 1H), 6.43 - 6.34 (m, 1H), 4.37-4.34 (m, 4H), 4.17 (t, J = 6.4 Hz, 2H), 3.59 (t, J = 4.6 Hz, 4H), 2.46 (t, J = 7.1 Hz, 2H), 2.39 (d, J = 4.6 Hz, 4H), 2.08 - 1.79 (m, 2H), 1.47 (d, J = 2.3 Hz, 4H); $^{13}$C NMR (101 MHz, DMSO-$d_6$) δ 168.7, 168.4, 160.9, 151.8, 149.6, 146.6, 138.2, 133.8, 129.8, 127.7, 123.4, 122.9, 115.6, 115.4, 107.9, 102.2, 67.1, 66.7, 64.4, 63.97, 55.2, 53.8, 32.3, 26.2, 15.7; MS: 661$[M+H]^+$.

Example 1. Preparation of the crystal form A-I of the mucate salt of the compound of formula (I)

Table 2. Characteristic peaks of mucate salt crystal form A-I

| Serial number | 2θ [°] | Interplanar spacing [Å] | Relative intensity [%] |
|---|---|---|---|
| Peak 1 | 3.47 | 25.45 | 37.18 |
| Peak 2 | 3.95 | 22.36 | 74.19 |
| Peak 3 | 5.54 | 15.95 | 100.00 |
| Peak 4 | 7.46 | 11.85 | 84.04 |
| Peak 5 | 8.46 | 10.45 | 30.77 |
| Peak 6 | 10.48 | 8.44 | 26.41 |
| Peak 7 | 10.87 | 8.14 | 40.65 |
| Peak 8 | 11.72 | 7.55 | 11.22 |
| Peak 9 | 14.08 | 6.29 | 19.10 |
| Peak 10 | 14.90 | 5.95 | 20.52 |
| Peak 11 | 15.28 | 5.80 | 22.05 |
| Peak 12 | 16.16 | 5.49 | 20.40 |
| Peak 13 | 16.58 | 5.35 | 97.69 |
| Peak 14 | 17.90 | 4.96 | 7.35 |
| Peak 15 | 20.29 | 4.38 | 9.82 |
| Peak 16 | 21.65 | 4.10 | 19.07 |
| Peak 17 | 22.35 | 3.98 | 14.52 |
| Peak 18 | 23.37 | 3.81 | 11.14 |
| Peak 19 | 24.53 | 3.63 | 5.51 |
| Peak 20 | 25.45 | 3.50 | 3.36 |
| Peak 21 | 27.35 | 3.26 | 7.94 |
| Peak 22 | 32.58 | 2.75 | 2.68 |

300 mg of the compound of formula (I) and mucic acid (acid-base molar ratio of 0.5:1) were suspended and stirred in acetone at room temperature for 4 days, and then 15 mg of the compound of formula (I) was added and the mixture was stirred at room temperature for another 2 days. The mixture was then dried under vacuum at room temperature to give the product.

$^1$H NMR results showed that the acid-base molar ratio in the sample was 0.5:1.

$^1$HNMR (400 MHz, DMSO-*d*$_6$) δ 10.34 (s, 1H), 10.02 (s, 1H), 8.42-8.41 (d, J = 4 Hz, 1H), 7.88-7.84(m, 1H), 7.66-7.62 (m, 2H), 7.47-7.44 (m, 1H), 7.26-7.13 (m, 3H), 7.07 (s, 1H), 6.40-6.39 (d, J = 4 Hz, 1H), 4.35 (s, 4H), 4.21 (s, 1H), 4.19-4.16 (t, 2H), 3.77 (s, 1H), 3.61-3.59(m, 4H), 2.43-2.41(m, 6H), 2.01-1.95 (m, 2H), 1.49-1.44 (m, 4H).

The crystal form was defined as crystal form A-I by X-ray powder diffraction detection. The XRPD diagram was shown in Fig. 1. The positions of its characteristic peaks were shown in Table 2 above, and the TGA and DSC spectra were shown in Figs. 2 and 3. The TGA/DSC results showed that the sample loses 1.1% of its weight upon heated to 150 °C, and has an endothermic peak at 207.3 °C (peak temperature).

Example 2. Preparation of the crystal form A-II of the p-toluenesulfonate salt of the compound of formula (I)

300 mg of the compound of formula (I) and p-toluenesulfonic acid (acid-base molar ratio of 1:1) were suspended and stirred in acetone at room temperature for 1 day, and then dried in vacuo at room temperature to give the product.

$^1$H NMR results showed that the acid-base molar ratio in the sample was 1.0:1.

$^1$H NMR (400 MHz, MeOD) δ 8.43-8.42 (d, J = 4 Hz, 1H), 7.86-7.82 (m, 1H), 7.70-7.68 (m, 2H), 7.60-7.56 (m, 2H), 7.43-7.40 (m, 1H), 7.25-7.20 (m, 3H), 7.12-7.07 (m, 3H), 6.57-6.56 (m, 1H), 4.44 (s, 4H), 4.37-4.34 (m, 2H), 3.97 (m, 4H), 3.44-3.38(m, 6H), 2.42-2.39 (m, 2H), 2.36 (s, 3H), 1.66 (s, 4H). (Active hydrogen on amide was not shown)

The crystal form was defined as crystal form A-II by X-ray powder diffraction detection. The XRPD pattern was shown in Fig. 4. The positions of its characteristic peaks were shown in Table 3 below. The TGA and DSC spectra were shown in Figs. 5 and 6. The TGA/DSC results showed that the sample loses 1.0% of its weight upon heated to 150 °C, and has an endothermic peak at 156.2 °C (peak temperature).

Table 3. Characteristic peaks of p-toluenesulfonate salt crystal form A-II

| Serial number | 2θ [°] | Interplanar spacing [Å] | Relative intensity [%] |
|---|---|---|---|
| Peak 1 | 5.30 | 16.67 | 87.09 |
| Peak 2 | 6.54 | 13.52 | 59.56 |
| Peak 3 | 7.97 | 11.10 | 97.83 |
| Peak 4 | 8.78 | 10.07 | 47.11 |
| Peak 5 | 10.52 | 8.41 | 35.27 |
| Peak 6 | 11.63 | 7.61 | 22.35 |
| Peak 7 | 11.81 | 7.50 | 27.69 |
| Peak 8 | 13.01 | 6.80 | 41.53 |
| Peak 9 | 13.54 | 6.54 | 14.07 |
| Peak 10 | 14.33 | 6.18 | 16.50 |
| Peak 11 | 14.52 | 6.10 | 16.95 |
| Peak 12 | 15.06 | 5.88 | 2.86 |
| Peak 13 | 15.86 | 5.59 | 56.25 |
| Peak 14 | 16.46 | 5.39 | 14.24 |
| Peak 15 | 17.54 | 5.06 | 25.01 |
| Peak 16 | 18.02 | 4.92 | 19.98 |
| Peak 17 | 18.25 | 4.86 | 15.41 |
| Peak 18 | 18.75 | 4.73 | 6.35 |
| Peak 19 | 19.34 | 4.59 | 24.35 |
| Peak 20 | 19.70 | 4.51 | 100.00 |
| Peak 21 | 19.94 | 4.45 | 44.94 |
| Peak 22 | 20.62 | 4.31 | 49.17 |
| Peak 23 | 21.06 | 4.22 | 17.90 |
| Peak 24 | 21.46 | 4.14 | 12.32 |
| Peak 25 | 22.50 | 3.95 | 32.24 |
| Peak 26 | 23.67 | 3.76 | 13.62 |
| Peak 27 | 24.02 | 3.71 | 13.38 |
| Peak 28 | 24.35 | 3.66 | 9.15 |
| Peak 29 | 25.12 | 3.54 | 5.86 |
| Peak 30 | 25.95 | 3.43 | 21.42 |
| Peak 31 | 26.45 | 3.37 | 7.80 |
| Peak 32 | 27.20 | 3.28 | 10.86 |
| Peak 33 | 27.87 | 3.20 | 5.01 |
| Peak 34 | 29.20 | 3.06 | 8.29 |
| Peak 35 | 30.15 | 2.96 | 5.93 |
| Peak 36 | 30.87 | 2.90 | 3.88 |
| Peak 37 | 31.83 | 2.81 | 3.19 |

Example 3. Preparation of p-toluenesulfonate salt crystal form B-III of the compound of formula (I)

Table 4 Characteristic peaks of p-toluenesulfonate salt crystal form B-III

| Serial number | 2θ [°] | Interplanar spacing [Å] | Relative intensity [%] |
|---|---|---|---|
| Peak 1 | 5.65 | 15.65 | 60.80 |
| Peak 2 | 6.79 | 13.02 | 20.54 |
| Peak 3 | 7.61 | 11.62 | 7.90 |

(continued)

| Serial number | 2θ [°] | Interplanar spacing [Å] | Relative intensity [%] |
|---|---|---|---|
| Peak 4 | 9.41 | 9.39 | 8.32 |
| Peak 5 | 11.22 | 7.89 | 24.41 |
| Peak 6 | 11.84 | 7.48 | 45.97 |
| Peak 7 | 16.23 | 5.46 | 24.72 |
| Peak 8 | 16.84 | 5.27 | 100.00 |
| Peak 9 | 18.51 | 4.79 | 15.33 |
| Peak 10 | 18.96 | 4.68 | 13.43 |
| Peak 11 | 19.87 | 4.47 | 22.65 |
| Peak 12 | 22.83 | 3.90 | 27.62 |
| Peak 13 | 25.61 | 3.48 | 8.14 |

About 50 mg of p-toluenesulfonate salt crystal form A-II product was suspended and stirred in water at 50°C for 1 day, filtered and dried at room temperature to give the product.

$^1H$ NMR results showed that the acid-base molar ratio in the sample was 0.9:1.

$^1H$ NMR (400 MHz, MeOD) δ 8.43-8.42 (d, J = 4 Hz, 1H), 7.86-7.82 (m, 1H), 7.70-7.68 (m, 2H), 7.60-7.56 (m, 2H), 7.43-7.40 (m, 1H), 7.25-7.20 (m, 3H), 7.12-7.07 (m, 3H), 6.57-6.56 (m, 1H), 4.44 (s, 4H), 4.37-4.34 (m, 2H), 3.97 (m, 4H), 3.44-3.38(m, 6H), 2.42-2.39 (m, 2H), 2.36 (s, 3H), 1.66 (s, 4H). (Active hydrogen on amide was not shown)

The crystal form was defined as crystal form B-III by X-ray powder diffraction detection. The XRPD pattern was shown in Fig. 7. The positions of its characteristic peaks were shown in Table 4 above. The TGA and DSC spectra were shown in Figs. 8 and 9. The TGA/DSC results showed that the sample loses 3.4% of its weight upon heated to 150 °C, and has two endothermic peaks at 124.4 and 139.3 °C (peak temperatures).

Example 4. Preparation of the di-p-toluenesulfonate salt crystal form A-IV of the compound of formula (I)

300 mg of the compound of formula (I) and p-toluenesulfonic acid (acid-base molar ratio of 2:1) were suspended and stirred in acetone at room temperature for 1 day, and then dried in vacuo at room temperature to give the product.

$^1H$ NMR results showed that the acid-base molar ratio in the sample was 2.0:1.

$^1H$ NMR (400 MHz, MeOD) δ 8.60-8.58 (d, J = 8 Hz, 1H), 7.97-7.93(m, 1H), 7.66-7.64 (m, 4H), 7.60-7.55 (m, 2H), 7.53-7.50 (m, 1H), 7.42-7.38 (m, 1H), 7.19-7.17 (m, 4H), 7.13-7.01 (m, 3H), 6.86-6.84 (m, 1H), 4.56-4.49 (m, 4H), 4.45-4.42 (m, 2H), 4.10 (m, 2H), 3.91(m, 2H), 3.72 (m, 2H), 3.53-3.49 (m, 2H), 3.22 (m, 2H), 2.46-2.43 (m, 2H), 2.34 (s, 6H), 1.61 (s, 4H). (Active hydrogen on amide was not shown)

The crystal form was defined as crystal form A-IV by X-ray powder diffraction detection. The XRPD pattern was shown in Fig. 10. The positions of its characteristic peaks were shown in Table 5 below. The TGA and DSC spectra were shown in Figs. 11 and 12. The TGA/DSC results showed that the sample loses 1.3% of its weight upon heated to 150 °C, and has an endothermic peak at 228.8 °C (peak temperature).

Table 5 Characteristic peaks of di-p-toluenesulfonate salt crystal form A-IV

| Serial number | 2θ [°] | Interplanar spacing [Å] | Relative intensity [%] |
|---|---|---|---|
| Peak 1 | 6.36 | 13.89 | 100.00 |
| Peak 2 | 9.45 | 9.36 | 6.32 |
| Peak 3 | 9.87 | 8.96 | 8.29 |
| Peak 4 | 11.50 | 7.70 | 6.97 |
| Peak 5 | 12.88 | 6.87 | 6.56 |
| Peak 6 | 13.60 | 6.51 | 16.30 |
| Peak 7 | 14.40 | 6.15 | 4.01 |
| Peak 8 | 16.00 | 5.54 | 8.55 |
| Peak 9 | 19.20 | 4.62 | 18.69 |
| Peak 10 | 20.26 | 4.38 | 8.29 |
| Peak 11 | 20.86 | 4.26 | 10.16 |

(continued)

| Serial number | 2θ [°] | Interplanar spacing [Å] | Relative intensity [%] |
|---|---|---|---|
| Peak 12 | 21.36 | 4.16 | 7.46 |
| Peak 13 | 22.59 | 3.94 | 6.51 |
| Peak 14 | 23.76 | 3.75 | 5.59 |
| Peak 15 | 26.73 | 3.34 | 5.07 |

Example 5. Preparation of crystal form A-V of the phosphate salt of the compound of formula (I)

300 mg of the compound of formula (I) and phosphoric acid (acid-base molar ratio of 1:1) were mixed in EtOH/$H_2O$ (19:1, v/v), and 25 mg of seed crystals (obtained by the following method: 50 mg of free state of the compound of formula (I) and phosphoric acid in an equal molar ratio were stirred in EtOH/$H_2O$ (19:1, v/v) at room temperature for 5 days, then filtered and dried at room temperature) were added. The mixture was suspended and stirred at room temperature for 2 days, and then dried under vacuum at room temperature to give the product. HPLC/IC results showed that the acid-base molar ratio of the sample was 0.8:1.

$^1$H NMR (400 MHz, MeOD) δ 8.40-8.39 (d, J = 4 Hz, 1H), 7.84-7.83 (d, J = 4 Hz, 1H), 7.60-7.56 (m, 2H), 7.41-7.38 (m, 1H), 7.23-7.18 (t, 1H), 7.12-7.07 (m, 2H), 6.52-6.50 (m, 2H), 4.42 (s, 4H), 4.32-4.29 (t, 2H), 3.89-3.87 (m, 4H), 3.09-3.02 (m, 6H), 2.32-2.25 (m, 2H), 1.66 (s, 4H). (Active hydrogen on amide was not shown)

The crystal form was defined as crystal form A-V by X-ray powder diffraction detection. The XRPD diagram was shown in Fig. 13. The positions of its characteristic peaks were shown in Table 6 below. The TGA and DSC spectra were shown in Figs. 14 and 15. The TGA/DSC results showed that the sample loses 7.1% of its weight upon heated to 150 °C, and has two endothermic peaks at 90.5 and 148.2 °C (peak temperatures).

Table 6. Characteristic peaks of phosphate salt crystal form A-V

| Serial number | 2θ [°] | Interplanar spacing [Å] | Relative intensity [%] |
|---|---|---|---|
| Peak 1 | 4.03 | 21.90 | 45.90 |
| Peak 2 | 5.68 | 15.57 | 89.31 |
| Peak 3 | 6.84 | 12.93 | 45.36 |
| Peak 4 | 7.56 | 11.70 | 100.00 |
| Peak 5 | 9.36 | 9.45 | 51.15 |
| Peak 6 | 11.30 | 7.83 | 36.19 |
| Peak 7 | 11.94 | 7.41 | 48.96 |
| Peak 8 | 12.56 | 7.05 | 25.32 |
| Peak 9 | 13.69 | 6.47 | 8.50 |
| Peak 10 | 16.17 | 5.48 | 29.73 |
| Peak 11 | 16.93 | 5.24 | 94.64 |
| Peak 12 | 19.76 | 4.49 | 32.86 |
| Peak 13 | 22.02 | 4.04 | 16.17 |
| Peak 14 | 23.01 | 3.87 | 32.49 |
| Peak 15 | 23.98 | 3.71 | 15.62 |
| Peak 16 | 25.19 | 3.54 | 9.08 |

**Assay Example 1.** Determination of the solubility of the salts and crystal forms of the present application in water

The dynamic solubility of the free state, the mucate salt crystal form A-I, the p-toluenesulfonate salt crystal form A-II, the di-p-toluenesulfonate salt crystal form A-IV and the phosphate salt crystal form A-V of the compound of formula (I) in water and three biological solvents was evaluated.

The solubility of each sample in four solvent systems of water, SGF, FaSSIF and FeSSIF (1, 2, 4 and 24 hours) was determined at 37 °C by rotating mixing (25 rpm) at a feed concentration of 10 mg/mL (40 mg of material was added to 4 mL of solvent). The samples at each time point were centrifuged and filtered (0.45 μm PTFE filter head), and the HPLC concentration and pH value of the filtrate were determined. The results of the solubility test were summarized in Table 7.

The results showed that in $H_2O$ and FaSSIF, the solubility of p-toluenesulfonate salt crystal form A-II, di-p-toluenesulfonate salt crystal form A-IV and phosphate salt crystal form A-V is significantly improved compared with the free state; in SGF, the solubility is relatively close; in FeSSIF, the solubility of the other four crystal forms is greatly improved compared with the free state.

Table 7 Summary of dynamic solubility test results

| Raw material | Solvent | 1 hour | | 2 hours | | 4 hours | | 24 hours | |
|---|---|---|---|---|---|---|---|---|---|
| | | S | pH | S | pH | S | pH | S | pH |
| Free state | $H_2O$ | <LOQ | 6.7 | <LOQ | 5.5 | ND | 6.7 | ND | 7.8 |
| | SGF | 5.7 | 3.6 | 5.8 | 3.7 | 5.7 | 3.7 | 5.7 | 3.8 |
| | FaSSIF | 0.0018 | 6.4 | 0.0019 | 6.4 | 0.0021 | 6.4 | 0.0025 | 6.4 |
| | FeSSIF | 0.64 | 4.9 | 0.71 | 5.0 | 0.71 | 5.0 | 0.76 | 5.0 |
| Mucate salt crystal form A-I | $H_2O$ | 0.11 | 4.5 | 0.11 | 4.4 | 0.13 | 4.8 | 0.11 | 4.7 |
| | SGF | 5.4 | 3.1 | 5.2 | 3.1 | 5.1 | 3.1 | 5.3 | 3.2 |
| | FaSSIF | 0.0036 | 6.3 | 0.0048 | 6.2 | 0.0042 | 6.2 | 0.0070 | 5.5 |
| | FeSSIF | 1.3 | 4.9 | 1.6 | 4.9 | 1.8 | 4.9 | 1.8 | 4.9 |
| p-Toluenesulfo nate salt crystal form A-II | $H_2O$ | 2.7 | 4.8 | 2.7 | 4.9 | 2.8 | 4.7 | 2.5 | 4.2 |
| | SGF | 6.8 | 2.3 | completely dissolved | | | | 6.3 | 2.3 |
| | FaSSIF | 0.073 | 6.1 | 0.066 | 6.1 | 0.059 | 6.1 | 0.072 | 5.1 |
| | FeSSIF | 1.8 | 5.0 | 2.0 | 5.0 | 1.5 | 5.0 | 0.84 | 5.0 |
| Di-p-toluenesulfon ate salt crystal form A-IV | $H_2O$ | 2.9 | 3.7 | 2.9 | 3.9 | 2.8 | 3.8 | 2.7 | 3.8 |
| | SGF | 4.5 | 1.9 | 4.6 | 1.9 | 4.3 | 1.9 | 4.2 | 1.9 |
| | FaSSIF | 0.035 | 5.6 | 0.080 | 5.2 | 0.16 | 5.0 | 0.20 | 5.0 |
| | FeSSIF | 1.6 | 4.9 | 1.8 | 4.8 | 1.4 | 4.8 | 1.0 | 4.9 |
| Phosphate salt crystal form A-V | $H_2O$ | 2.5 | 4.5 | 2.6 | 4.5 | 2.4 | 4.5 | 2.5 | 4.4 |
| | SGF | 6.9 | 2.7 | completely dissolved | | | | 6.4 | 2.7 |
| | FaSSIF | 0.028 | 5.8 | 0.030 | 5.8 | 0.024 | 5.8 | 0.080 | 5.3 |
| | FeSSIF | 1.5 | 4.9 | 1.4 | 5.0 | 1.3 | 5.0 | 0.20 | 5.0 |
| LOQ: 0.60 μg/mL; ND: Not Detected | | | | | | | | | |

The equilibrium solubility of p-toluenesulfonate salt crystal form A-II and crystal form B-III in water was also evaluated. ~10/8 mg of material was weighed into an HPLC vial, 1.0/0.8 mL of water was added, and the mixture was suspended and stirred at room temperature for 24 hours. The sample was centrifuged and filtered, and the filtrate was tested by HPLC. The solubility results are shown in Table 8, and the results showed that the p-toluenesulfonate salt crystal form A-II has a higher solubility than that of the crystal form B-III.

Table 8. Summary of equilibrium solubility results

| Raw material | Solvent | Solubility (mg/mL) |
|---|---|---|
| p-Toluenesulfonate salt crystal form **A-II** | $H_2O$ | 5.1 |
| p-Toluenesulfonate salt crystal form **B-III** | $H_2O$ | 1.3 |

Assay Example 2. Study on the hygroscopicity of the salts and crystal forms of the present application

The hygroscopicity of the free compound of formula (I), mucate salt crystal form A-I, p-toluenesulfonate salt crystal forms A-II and B-III, di-p-toluenesulfonate salt crystal form A-IV and phosphate salt crystal form A-V was evaluated by dynamic moisture sorption instrument (DVS). Starting from 0 % relative humidity (0%RH), the test collected the mass change percentage of the samples under constant temperature of 25 °C with humidity changes (0%RH-95%RH-0%RH). The moisture adsorption of the 6 test samples at 25 °C/80%RH is shown in Table 9 below. The results showed that the free state of the compound of formula (I) is almost non-hygroscopic, its p-toluenesulfonate salt crystal form A-II is slightly hygroscopic, p-toluenesulfonate salt crystal form B-III, mucate salt crystal form A-I, di-p-toluenesulfonate salt crystal form A-IV and phosphate salt crystal form A-V are hygroscopic, and the crystal form of each sample remains unchanged before and after the DVS test.

Table 9 Hygroscopicity evaluation results

| Salt type | Moisture adsorption (25 °C/80%RH) | Hygroscopicity | Whether the crystal form changes after DVS test |
|---|---|---|---|
| Free state | 0.11% | Almost no hygroscopicity | no |
| Mucate salt crystal form A-I | 2.09% | Hygroscopic | no |
| p-Toluenesulfonate salt crystal form A-II | 0.52% | Slightly hygroscopic | no |
| p-Toluenesulfonate salt crystal form B-III | 3.13% | Hygroscopic | no |
| Di-p-toluenesulfonate salt crystal form A-IV | 3.70% | Hygroscopic | no |
| Phosphate salt crystal form A-V | 6.43% | Hygroscopic | no |

**Assay Example 3.** Stability study of the salts and crystal forms of the present application

The following 6 test samples were placed under 25 °C / 60% RH and 40 °C / 75% RH conditions for 1 week, and then the physical and chemical stability of the samples was tested by XRPD and HPLC. The test data are listed in the following Table 10. The results showed that the 6 samples did not undergo apparent degradation upon placed under 25 °C / 60% RH and 40 °C / 75% RH conditions for 1 week, the chemical stability was good, and the crystal form did not change.

At the same time, after the p-toluenesulfonate salt crystal form A-II and crystal form B-III solids were placed at 80 °C for 24 hours, the physical and chemical stability of the samples were tested by XRPD and HPLC. The results showed that the p-toluenesulfonate salt crystal form A-II and crystal form B-III did not undergo crystal form changes or chemical degradation under such high temperature conditions.

Table 10. Summary of solid-state stability evaluation

| Starting sample | Condition | Time | Purity (area %) | Purity/initial purity (%) | Change of Crystal form |
|---|---|---|---|---|---|
| Free state | Starting | - | 99.44 | - | no |
| | 25 °C/60%RH | 1 week | 99.36 | 99.9 | no |
| | 40 °C/75%RH | 1 week | 99.39 | 99.9 | no |
| Mucate salt crystal form A-I | Starting | - | 99.56 | - | no |
| | 25 °C/60%RH | 1 week | 99.54 | 100.0 | no |
| | 40 °C/75%RH | 1 week | 99.56 | 100.0 | no |
| p-Toluenesulfonate | Starting | - | 99.44 | - | no |
| | 80 °C | 24 hours | 99.42 | 99.9 | no |
| salt crystal form A-II | 25 °C/60%RH | 1 week | 99.52 | 100.1 | no |
| | 40 °C/75%RH | 1 week | 99.44 | 100.0 | no |
| p-Toluenesulfonate salt crystal form B-III | Starting | - | 99.79 | - | - |
| | 80 °C | 24 hours | 99.56 | 99.8 | no |
| | 25 °C/60%RH | 1 week | 99.68 | 99.9 | no |
| | 40 °C/75%RH | 1 week | 99.63 | 99.8 | no |
| Di-p-toluenesulfonate salt crystal form A-IV | Starting | - | 99.18 | - | no |
| | 25 °C/60%RH | 1 week | 99.19 | 100.0 | no |
| | 40 °C/75%RH | 1 week | 99.25 | 100.1 | no |
| Phosphate salt crystal form A-V | Starting | - | 99.23 | - | no |
| | 25 °C/60%RH | 1 week | 99.13 | 99.9 | no |
| | 40 °C/75%RH | 1 week | 99.13 | 99.9 | no |

**Long-term stability study**

p-Toluenesulfonate salt crystal form A-II of the compound of formula (I) was placed at 25 °C/60% RH for 6 months to investigate its long-term stability. The test data are listed in the following Table 11. The results showed that the p-toluenesulfonate salt crystal form A-II of the compound of formula (I) did not undergo apparent degradation and had good physical and chemical stability, and the crystal form did not change upon placed at 25 °C/60% RH for 6 months.

Table 11. Summary of long-term stability evaluation of the p-toluenesulfonate salt crystal form A-II of the compound of formula (I)

| Test conditions | Sampling time | Appearance | Content | Total impurities | Moisture | Crystal form |
|---|---|---|---|---|---|---|
| - | Day 0 | Off-white powder | 100.2% | 0.37% | 0.53% | No change |
| 25°C/60%RH, Simulating factory packaging | 3 months | Off-white powder | 100.0% | 0.38% | 0.31% | No change |
| | 6 months | Off-white powder | 100.5% | 0.36% | 0.59% | No change |

**Assay Example 4.** Study on the kinase activity of the salts and crystal forms of the present application

The present application studied the biological kinase activity of the p-toluenesulfonate salt crystal form A-II of the free compound prepared in Example 2, and found that in addition to VEGFR-2 and c-MET, it can also effectively inhibit the activity of tyrosine kinases such as PDGFRa, RET, c-KIT, TRK-A, TRK-B, and AXL.

The assay used the Mobility Shift Assay method. The starting concentration of the test substance was 2.5 μM, which was serially diluted 3-fold to give a total of 10 concentrations, in duplicate.

Table 12. Reagent information of kinase activity assay

| Name | Brand | Catalog NO. | Batch NO. |
|---|---|---|---|
| AXL | Carna | 08-107 | 06CBS-3408 |
| KDR(VEGFR2) | Carna | 08-191 | 07CBS-0540 |
| ekIT | eurofins | 14-559M | 1980267-C |
| TRK-A | Carna | 08-186 | 08CBS-0292 |
| TRK-B | Carna | 08-187 | 12CBS-0461Q |
| PDGFRa | BPS | 40260 | 1001 |
| MET | Carna | 08-151 | 10CBS-1118P |
| RET | Carna | 08-159 | 13CBS-0134E |
| P2 | GL Biochem | 112394 | P131014-XP112394 |
| P22 | GL Biochem | 112393 | P180116-MJ112393 |
| ATP | Sigma | A7699-1G | 987-65-5 |
| DMSO | Sigma | D2650 | 474382 |
| Staurosporine | MCE | HY-15141 | 21226 |
| Fluorescein-Poly GAT | Invitrogen | PV3611 | No 374293B |
| LanthaScreen® Tb-PY20 Antibody Kit | Invitrogen | PV3552 | 1508164B |
| HEPES, pH7.5 | Gibco | 11344-041 | 1653630 |
| Brij-35 solution | Sigma | B4184 | 018K61251 |
| EDTA | Gibco | 15575-038 | 846901 |
| MgC12 | Sigma | M2670-500g | BCBM7703V |
| DTT | Sigma | D0632-10G | SLBK4951V |
| EGTA | Sigma | E3889-25G | 129K54001V |
| 96 well plate | Corning | 3365 | 22008026 |
| 384 well plate | Corning | 3573 | 19714026 |
| 384 well Echo plate | Labcyte | PP-0200 | 0006386422 |

(continued)

| Name | Brand | Catalog NO. | Batch NO. |
|---|---|---|---|
| 384 well plate | Corning | 4512 | 13315017 |

Table 13. Instrument information of kinase activity test

| Chinese name | English name | Model | Supplier | Instrument No. |
|---|---|---|---|---|
| 激酶检测仪/微流控系统 | Caliper EZ Reader | EZ Reader II | Perkin Elmer | DS1152N0107 |
| | (No.2) | | | |
| 激酶检测仪/微流控系统 | Caliper EZ Reader (No.5) | EZ Reader II | Perkin Elmer | DS1652N0163 |
| 自动微孔移液器 | Precision | PRC384U | BioTek | 214145 |
| 生化培养箱 | Biochemical incu-bator | SPX-100B-Z | Shanghai Boxun | / |
| 离心机 | Centrifuge | 5810R | Eppendorf | 5811L941179 |
| 多标记检测分析仪 | Envision | 2104 Multilabel Reader | Perkin Elmer | 1041604 |
| 超声波纳升液体处理系统 | Echo | Echo550 | Labcyte | / |

Assay methods

1. Preparation of 1x kinase buffer and stop solution

1.1 1x kinase buffer

50 mM HEPES, pH 7.5
0.0015% Brij-35

1.2 Stop solution

100 mM HEPES, pH 7.5
0.015% Brij-35
0.2% Coating Reagent #3
50 mM EDTA

2. Compound preparation

1) Compound dilution

The p-toluenesulfonate salt of the free compound prepared in Example 2 was used as the test compound. The starting concentration of the test was 2.5 μM, and the concentration was prepared to 50 times, that is, 125 μM. 158 μl of 100% DMSO was added to the second well of a 96-well plate, and then 2 μl of 10 mM compound solution was added to prepare a 125 μM compound solution. 60 μl of 100% DMSO was added to the other wells. 30 μl of the compound was taken out from the second well and added to the third well, and the 3-fold dilution was made successively to give a total of 10 dilutions. Dilution instrument: automatic microwell pipette (Precision PRC384U)

2) Transferring 5 times the compound to the reaction plate

(1) 10 μl was taken out from each well of the 96-well plate with 50-fold compound concentration and transferred to another 96-well plate. 90 μl of kinase buffer was added to prepare a 5-fold compound concentration.
(2) 5 μl was taken out from the 96-well plate with 5-fold compound concentration and transferred to a 384-well reaction plate. For example, the compound in well A1 of the 96-well plate was transferred to wells A1 and A2 of the 384-well plate, the compound in well A2 of the 96-well plate was transferred to wells A3 and A4 of the 384-well plate, and so on.

3. Kinase reaction

1) Preparation of 2.5 times enzyme solution

Kinase was added to 1x kinase buffer to make a 2.5 times enzyme solution.

2) Addition of enzyme solution to the 384-well plate

(1) 5 μl of 5x compound dissolved in 10% DMSO was already in the 384-well reaction plate.
(2) 10 μl of 2.5x enzyme solution was added to the 384-well reaction plate and 10 μl of kinase buffer was added to the negative control wells.
(3) Incubation at room temperature for 10 minutes.

3) Preparation of 2.5 times the substrate solution

FAM-labeled peptide and ATP (ATP concentration see Appendix) were added to 1x kinase buffer to form a 2.5x substrate solution.

4) Addition of substrate solution to the 384-well plate.

10 μl of 2.5x substrate solution was added to the 384-well reaction plate and centrifuged at 1000 rpm for 1 minute.

5) Kinase reaction and termination

(1) The plate was incubated at 28°C for 60 minutes (Biochemical incubator model: SPX-100B-Z).
(2) 30 μl of stop solution was added to the 384-well reaction plate to terminate the reaction and centrifuged at 1000 rpm for 1 minute.

4. Data reading on Caliper EZ Reader II

The data of conversion rate was read on Caliper EZ Reader II.

5. Calculation of inhibition rate

1) The conversion rate data was copied from Caliper EZ Reader II.
2) The conversion rate was converted to inhibition rate data, where max refers to the conversion rate of the DMSO control and min refers to the conversion rate of the no enzyme activity control.

$$\text{Percent inhibition} = (\text{max-conversion}) / (\text{max-min})*100.$$

3) Fitting of $IC_{50}$ values using XLFit excel add-in version 5.4.0.8

$$\text{Fitting formula: } Y=Bottom + (Top-Bottom) / (1+(IC_{50}/X)^\wedge HillSlope)$$

Table 14. Kinase assay conditions

| Kinase | Substrate | ATP concentration (μM) |
|---|---|---|
| **KDR** | P22 | 92 |
| **PDGFRa** | P22 | 134 |
| **AXL** | P22 | 81 |
| **TRK-A** | P22 | 415 |
| **TRK-B** | P22 | 90 |
| **MET** | P2 | 26 |
| **RET** | P2 | 23 |
| **KIT** | P22 | 6 |

The study found that the $IC_{50}$ of the test substances for the inhibition of the above tyrosine kinases was less than or equal to 10.0 nM.

**Assay Example 5.** Study on the efficacy of different salts and the free compound of the present application on tumor-bearing mice

In this assay, the free compound of formula (I) of the present application and different salts of the compound prepared in Examples 1, 2 and 5 were orally administered to a nude mouse tumor-bearing model of human lung cancer cells EBC-1 to study their effects on tumor growth.

**Table 15. Instruments for drug efficacy assays in tumor-bearing mice:**

| Instrument name | Brand | Model |
|---|---|---|
| Electronic balance (mouse) | Ohaus Instruments (Changzhou) Co., Ltd. | Scout SE-SE202F |
| Single channel pipette | Mettler Toled | L-200XLS+ |
| Centrifuge | eppendort | Cantnfuge 5424 R |
| Clean bench | ESCO | ACB-6A1 |
| Vortex mixer | Northern Tongzheng | HQ-60W |
| Transfer window | Lingyun Boji (Beijing) Technology Co., Ltd. | unknown |
| IVC independent ventilation animal cage | Lingyun Boji (Beijing) Technology Co., Ltd. | LYBJ-IVCS |
| Electronic balance (mouse) | Changshu Shuangjie Testing Instrument Factory | T1000 |

**Table 16. Assay animals and cells:**

| Species | Strain | Gender | Quantity | Age | Weight | Source | Animal Certificate Number |
|---|---|---|---|---|---|---|---|
| Mouse | Balb/c nude | female | 55 | 6-8 weeks | 18-20g | Vital River Nanjing | 110011211100936675 |

(continued)

| Species | Strain | Gender | Quantity | Age | Weight | Source | Animal Certificate Number |
|---|---|---|---|---|---|---|---|
| EBC-1 | - | - | - | - | - | Science and Technology | - |

**Reagents:** RPMI1640 (ThermoFisher, catalog number C11875500BT); fetal bovine serum (Hyclone, catalog number SV30087.03); 0.25% trypsin-EDTA (ThermoFisher, catalog number 25200072); penicillin-streptomycin (Hyclone, catalog number SV30010); DSMO (Life Science, catalog number 0231-500ML); Solutol (Sigma, 70142-34-6-1 kg)

Preparation of test compounds: an appropriate weight of the test compound (including the free form of the compound of formula (I) and different salts of the compound prepared in Examples 1, 2 and 5) was weighed, completely suspended/dissolved in an appropriate volume of 0.1% sodium carboxymethyl cellulose, stirred and vortexed to give a uniform solution or suspension.

**Methods:** All assays were authorized by the Animal Welfare Committee. EBC-1 in the logarithmic growth phase was inoculated into immune-deficient nude mice (BALB/c nude, female, 6-8 weeks old, weighing 18 ± 2g), subcutaneously on the right back, with a cell inoculation amount of $5\times 10^6$ / mouse. After the tumor grew to 150-200 $mm^3$, the animals were randomly divided into groups, 6 mice per group. The dose of each test substance was set at 15 mg/kg and 7.5 mg/kg (both based on the concentration of the effective formula (I) compound), and administered once a day for about 3 weeks. The test compounds were freshly prepared each day before use. During the assay, the tumor diameter was measured twice a week, and the mice were weighed at the same time. The calculation formula of tumor volume (TV) is: TV = $1/2\times a\times b^2$, where a and b represent length and width, respectively. The relative tumor volume (RTV) was calculated based on the measurement results, and the calculation formula is: RTV = Vt/V0, wherein V0 is the tumor volume measured at the time of cage grouping and dosing initiation (i.e., d0), and Vt is the tumor volume at each measurement. The evaluation index of antitumor activity is the relative tumor proliferation rate T/C (%), calculated as follows: T/C (%) = (TRTV / CRTV) × 100%, TRTV: RTV of the treatment group; CRTV: RTV of the negative control group. T and C represent the average tumor volume at a certain time point in the drug administration group and the control group, respectively.

**Results:** The assay results are shown in Table 17 and Figs. 16-19. The different salts of the compound of formula (I) and its free compound all showed certain anti-tumor effects at doses of 15 mg/kg and 7.5 mg/kg, and the effect was significantly enhanced at a dose of 15 mg/kg. At the same time, compared with the free compound, the three different salts all showed better anti-tumor efficacy, especially the p-toluenesulfonate salt of the compound of formula (I) had a more significant effect.

Table 17: T/C (%) of the compounds in the examples of the present application in the nude mouse tumor model of human lung cancer EBC-1

| | Dose | **3(d)** | **7(d)** | **10(d)** | **14(d)** | **18(d)** | **21(d)** |
|---|---|---|---|---|---|---|---|
| Free compound | 15mg/kg 10ul/g | 88.8 | 76.9 | 80.4 | 66.4 | 72.2 | 64.60 |
| | 7.5mg/kg 10ul/g | 80.2 | 68.3 | 77.9 | 69.3 | 81.3 | 70.68 |
| Example 1 | 15mg/kg 10ul/g | 66.3 | 40.4 | 30.9 | 25.3 | 25.4 | 19.41 |
| | 7.5mg/kg 10ul/g | 71.1 | 60.2 | 49.9 | 46.9 | 47.4 | 43.73 |
| Example 2 | 15mg/kg 10ul/g | 67.9 | 37.3 | 25.4 | 17.5 | 16.0 | 12.09 |
| | 7.5mg/kg 10ul/g | 68.3 | 61.3 | 47.4 | 40.3 | 37.7 | 33.1 |
| Example 5 | 15mg/kg 10ul/g | 76.0 | 49.9 | 43.8 | 37.1 | 33.0 | 26.2 |
| | 7.5mg/kg 10ul/g | 97.1 | 80.6 | 76.3 | 68.2 | 66.5 | 58.8 |

The above is an alternative embodiment of the present disclosure. It should be pointed out that for ordinary technicians in this technical field, without departing from the principles of the present disclosure, the embodiments of the present disclosure may also be improved and modified. These improvements and modifications should also be regarded as falling within the scope of protection of the present disclosure.

## Claims

1. A pharmaceutically acceptable salt of a compound represented by formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide,

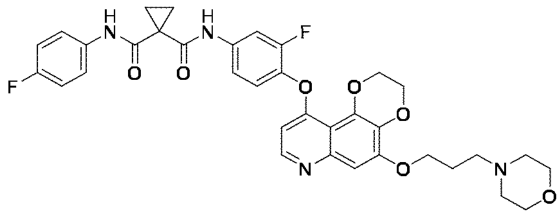

Formula (I),

wherein the pharmaceutically acceptable salt is selected from inorganic salts and organic salts, alternatively a p-toluenesulfonate salt, a mucate salt, and a phosphate salt.

2. The pharmaceutically acceptable salt according to claim 1, wherein the pharmaceutically acceptable salt is a salt of N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide and p-toluenesulfonic acid in a chemical ratio of 1:1 or 1:2.

3. A method for preparing a pharmaceutically acceptable salt according to claim 1, comprising the step of subjecting N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide to a salt-forming reaction with an inorganic acid or an organic acid, alternatively with p-toluenesulfonic acid, mucic acid or phosphoric acid.

4. The preparation method according to claim 3, wherein the salt-forming reaction is carried out in a solvent selected from water, alcohol solvents, halogenated hydrocarbon solvents, ketone solvents, ether solvents, nitrile solvents, ester solvents, amide solvents, aliphatic hydrocarbon solvents, alicyclic hydrocarbon solvents, aromatic hydrocarbon solvents, a mixed solvent of alcohol solvents and water, a mixed solvent of ketone solvents and water, a mixed solvent of alcohol solvents and ether solvents, a mixed solvent of halogenated hydrocarbon solvents and nitrile solvents, a mixed solvent of amide solvents and water, or a mixed solvent of nitrile solvents and water; the ketone solvent is alternatively acetone, the alcohol solvent is alternatively methanol, ethanol or isopropanol, the ether solvent is alternatively diethyl ether, methyl tert-butyl ether, tetrahydrofuran, 2-methyltetrahydrofuran or dioxane, the halogenated hydrocarbon solvent is alternatively dichloromethane or chloroform, the nitrile solvent is alternatively acetonitrile, the ester solvent is alternatively ethyl acetate, isopropyl acetate or butyl acetate, the amide solvent is alternatively N,N-dimethylformamide or N,N-dimethylacetamide, the aliphatic hydrocarbon solvent is alternatively n-heptane, the alicyclic hydrocarbon solvent is alternatively cyclohexane, the aromatic hydrocarbon solvent is alternatively toluene, xylene or isopropylbenzene, the mixed solvent of the alcohol solvent and the ether solvent is alternatively a mixed solvent of diethyl ether and methanol, the mixed solvent of the halogenated hydrocarbon solvent and the nitrile solvent is alternatively a mixed solvent of dichloromethane and acetonitrile, the mixed solvent of the alcohol solvent and water is alternatively a mixed solvent of methanol and water or ethanol and water, the mixed solvent of the ketone solvent and water is alternatively a mixed solvent of acetone and water, and the mixed solvent of the amide solvent and water is alternatively a mixed solvent of N,N-dimethylformamide and water.

5. The method for preparing a pharmaceutically acceptable salt according to claim 3, comprising the step of forming a salt from N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide and p-toluenesulfonic acid in a chemical ratio of 1:1 or 1:2.

6. A crystal form A-I of a mucate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 3.95, 5.54, 7.46, 10.87, and 16.58, wherein the error range of 2θ angle of each characteristic peak is ±0.2;

alternatively, there are characteristic peaks at 2θ angles of 3.47, 3.95, 5.54, 7.46, 8.46, 10.48, 10.87, 14.90, 15.28,

16.16, and 16.58, wherein the error range of 2θ angle of each characteristic peak is ±0.2;
yet alternatively, there are characteristic peaks at 2θ angles of 3.47, 3.95, 5.54, 7.46, 8.46, 10.48, 10.87, 11.72, 14.08, 14.90, 15.28, 16.16, 16.58, 17.90, 20.29, 21.65, 22.35, 23.37, 24.53, 25.45, 27.35, and 32.58, wherein the error range of 2θ angle of each characteristic peak is ±0.2.

**7.** A crystal form A-II of a p-toluenesulfonate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 5.30, 6.54, 7.97, 8.78, 13.01, 15.86, 19.70, 19.94, and 20.62, wherein the error range of 2θ angle of each characteristic peak is ±0.2;

alternatively, there are characteristic peaks at 2θ angles of 5.30, 6.54, 7.97, 8.78, 10.52, 11.63, 11.81, 13.01, 15.86, 17.54, 19.34, 19.70, 19.94, 20.62, 22.50, and 25.95, wherein the error range of 2θ angle of each characteristic peak is ±0.2;
yet alternatively, there are characteristic peaks at 2θ angles of 5.30, 6.54, 7.97, 8.78, 10.52, 11.63, 11.81, 13.01, 13.54, 14.33, 14.52, 15.06, 15.86, 16.46, 17.54, 18.02, 18.25, 18.75, 19.34, 19.70, 19.94, 20.62, 21.06, 21.46, 22.50, 23.67, 24.02, 24.35, 25.12, 25.95, 26.45, 27.20, 27.87, 29.20, 30.15, 30.87, and 31.83, wherein the error range of 2θ angle of each characteristic peak is ±0.2.

**8.** A crystal form A-IV of a di-p-toluenesulfonate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 6.36, 13.60, and 19.20, wherein the error range of 2θ angle of each characteristic peak is ±0.2;

alternatively, there are characteristic peaks at 2θ angles of 6.36, 9.87, 11.50, 13.60, 16.00, 19.20, 20.26, 20.86, and 21.36, wherein the error range of 2θ angle of each characteristic peak is ±0.2;
yet alternatively, there are characteristic peaks at 2θ angles of 6.36, 9.45, 9.87, 11.50, 12.88, 13.60, 14.40, 16.00, 19.20, 20.26, 20.86, 21.36, 22.59, 23.76, and 26.73, wherein the error range of 2θ angle of each characteristic peak is ±0.2.

**9.** A crystal form A-V of a phosphate salt of the compound of formula (I) N-(3-fluoro-4-((5-(3-morpholinopropoxy)-2,3-dihydro-[1,4]dioxano[2,3-f]quinolin-10-yl)oxy)phenyl)-N-(4-fluorophenyl)cyclopropane-1,1-dicarboxamide, wherein in its X-ray powder diffraction pattern, there are characteristic peaks at 2θ angles of 5.68, 7.56, and 16.93, wherein the error range of 2θ angle of each characteristic peak is ±0.2;

alternatively, there are characteristic peaks at 2θ angles of 4.03, 5.68, 6.84, 7.56, 9.36, 11.30, 11.94, 16.93, 19.76, and 23.01, wherein the error range of 2θ angle of each characteristic peak is ±0.2;
yet alternatively, there are characteristic peaks at 2θ angles of 4.03, 5.68, 6.84, 7.56, 9.36, 11.30, 11.94, 12.56, 13.69, 16.17, 16.93, 19.76, 22.02, 23.01, 23.98, and 25.19, wherein the error range of 2θ angle of each characteristic peak is ±0.2.

**10.** A pharmaceutical composition comprising the pharmaceutically acceptable salt according to any one of claims 1 to 2 or the crystal form according to any one of claims 6 to 9, and one or more pharmaceutically acceptable carriers or excipients.

**11.** The pharmaceutical composition according to claim 10, wherein the pharmaceutical composition further comprises one or more additional therapeutic agents.

**12.** Use of the pharmaceutically acceptable salt according to any one of claims 1 to 2, the crystal form according to any one of claims 6 to 9, or the pharmaceutical composition according to claims 10 to 11 in the manufacture of a medicament for treating diseases related to tyrosine kinases VEGFR-2, c-MET, c-KIT, PDGFRa, RET, AXL, or NTRK.

**13.** The use according to claim 12, wherein the disease is cancer or an autoimmune disease, in particular ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary tract carcinosarcoma, or cholangiocarcinoma.

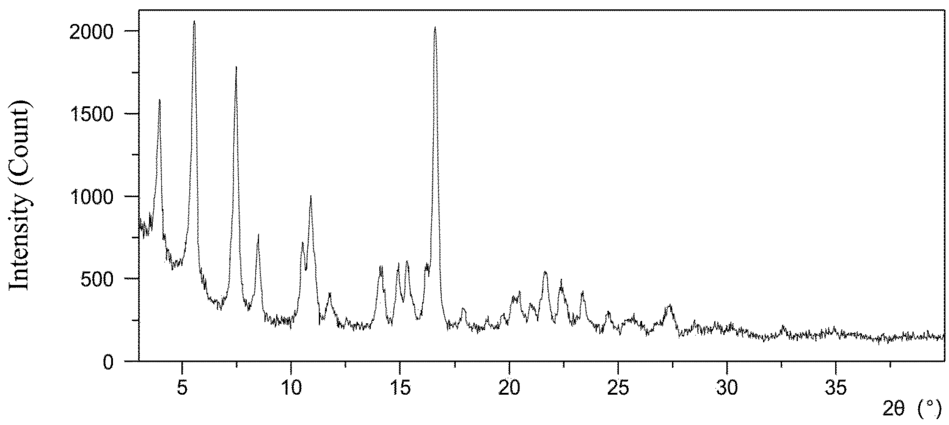


Fig. 1

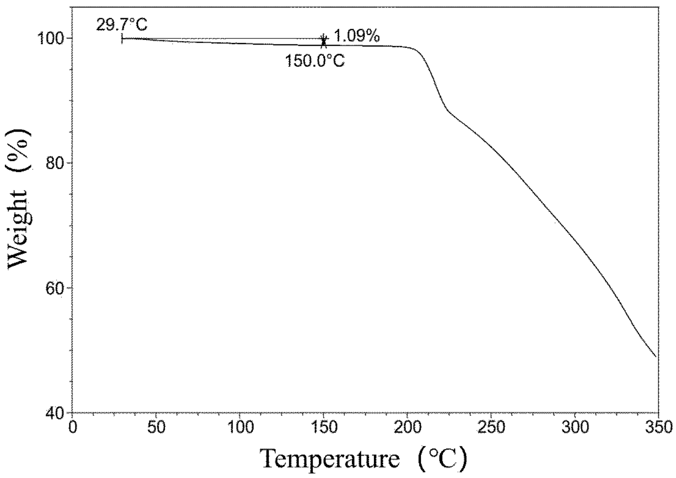


Fig. 2

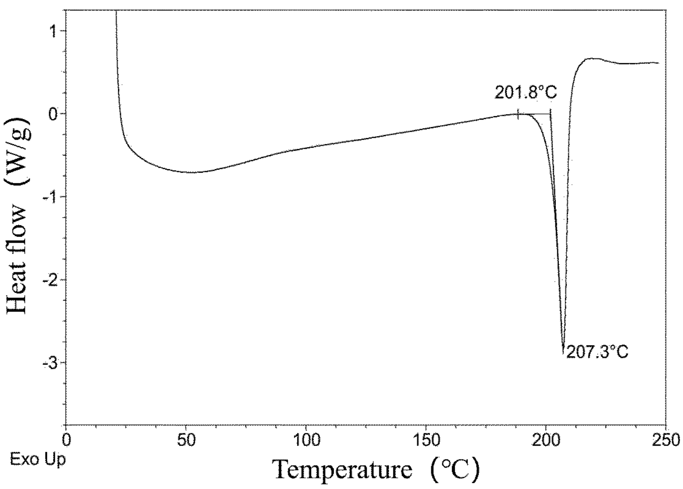


Fig. 3

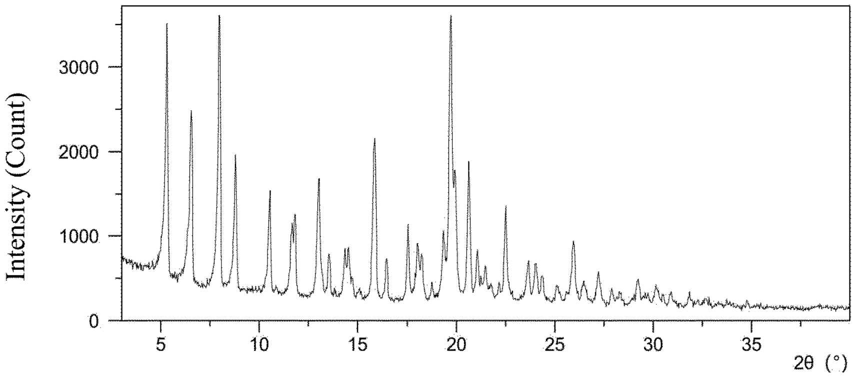


Fig. 4

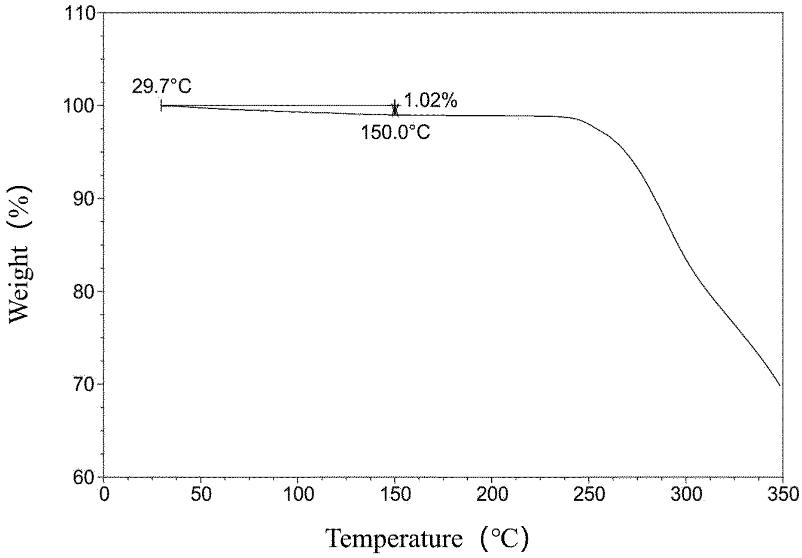


Fig. 5

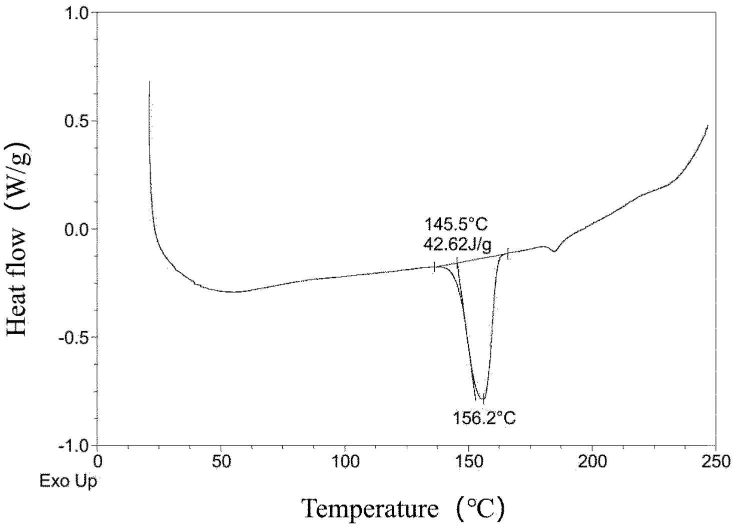


Fig. 6

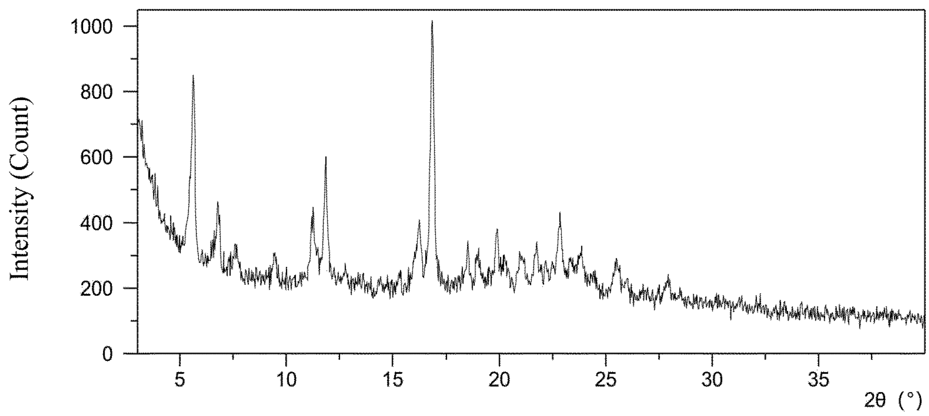


Fig. 7

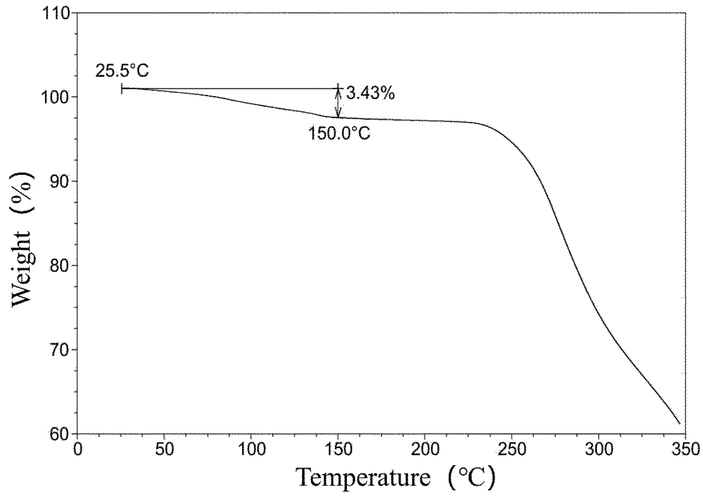


Fig. 8

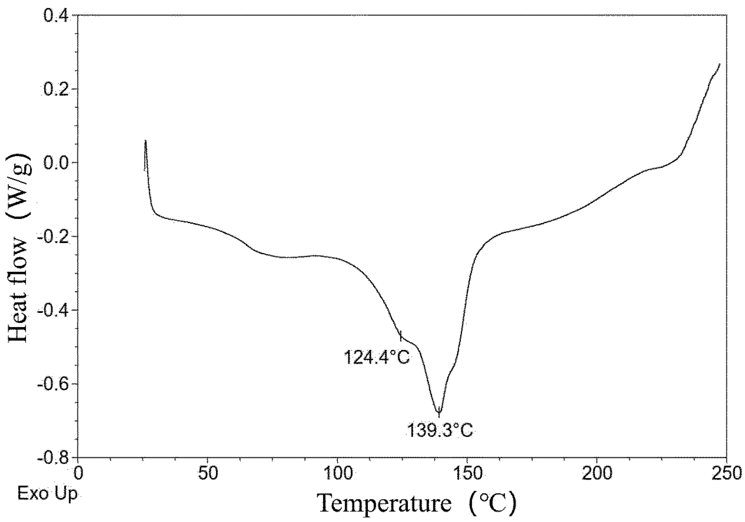


Fig. 9

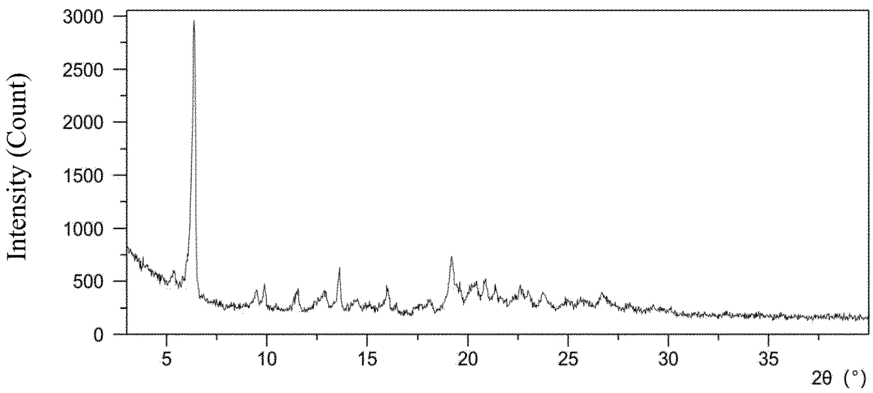


Fig. 10

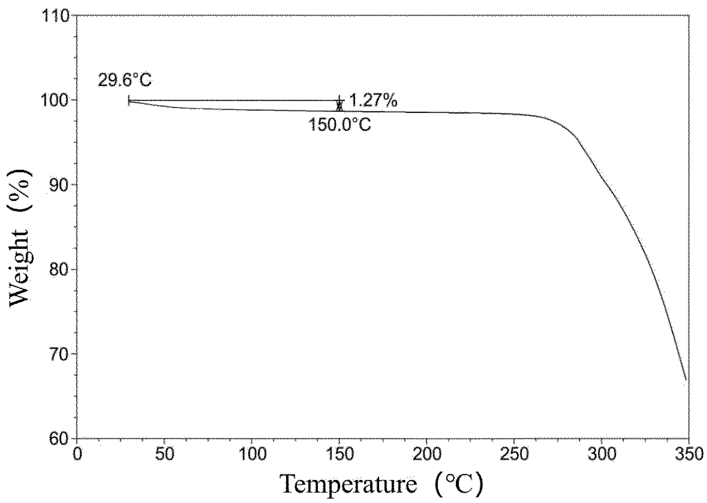


Fig. 11

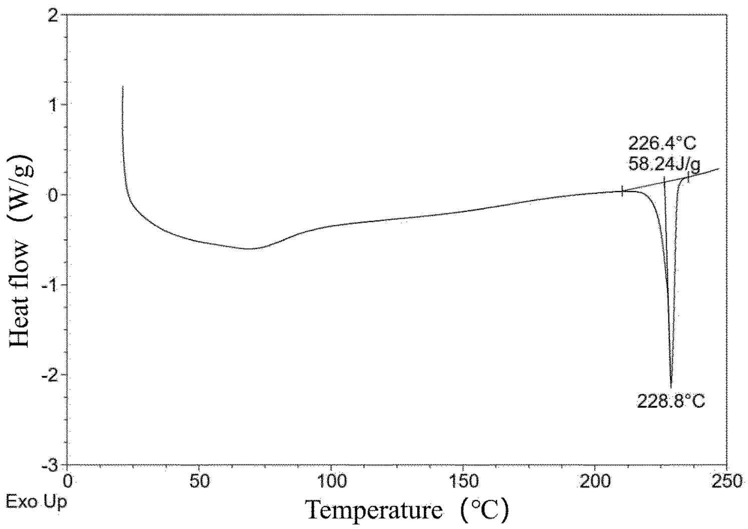


Fig. 12

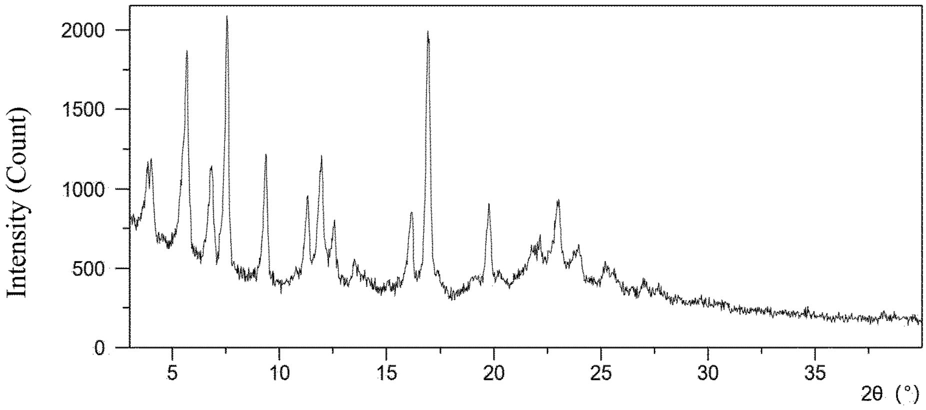


Fig. 13

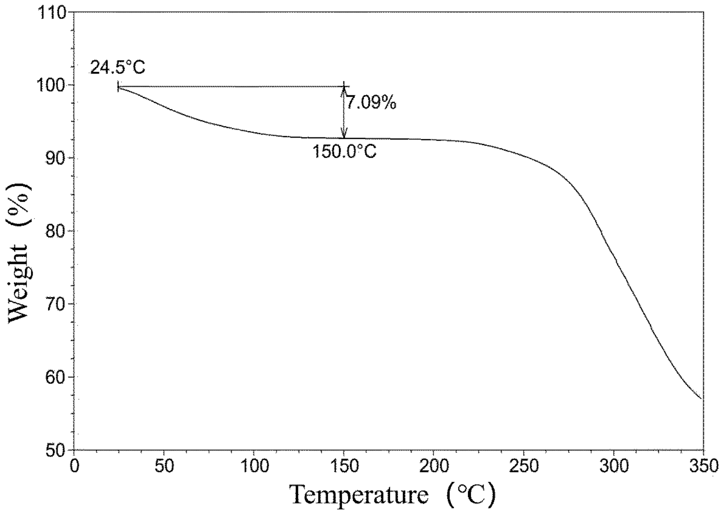


Fig. 14

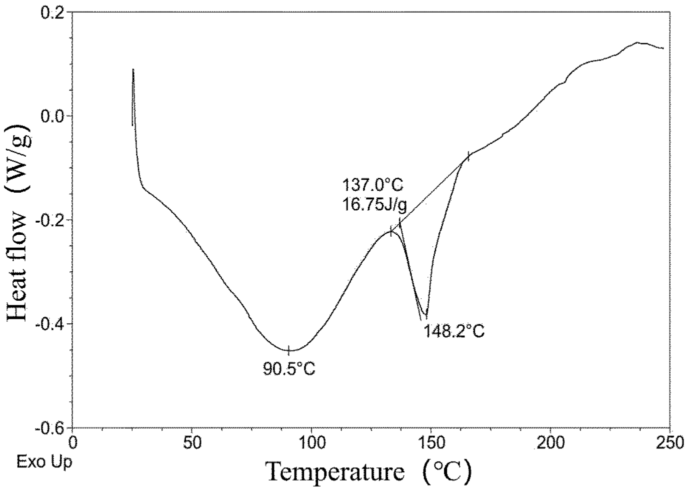


Fig. 15

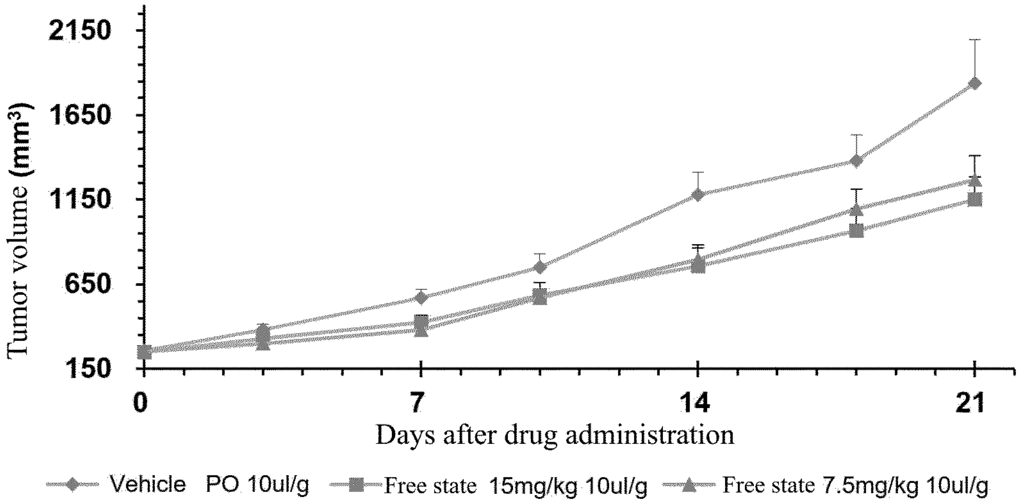

2150
1650
1150
650
150
Tumor volume (mm³)
0
7
14
21
Days after drug administration
Vehicle PO 10ul/g
Free state 15mg/kg 10ul/g
Free state 7.5mg/kg 10ul/g


Fig. 16

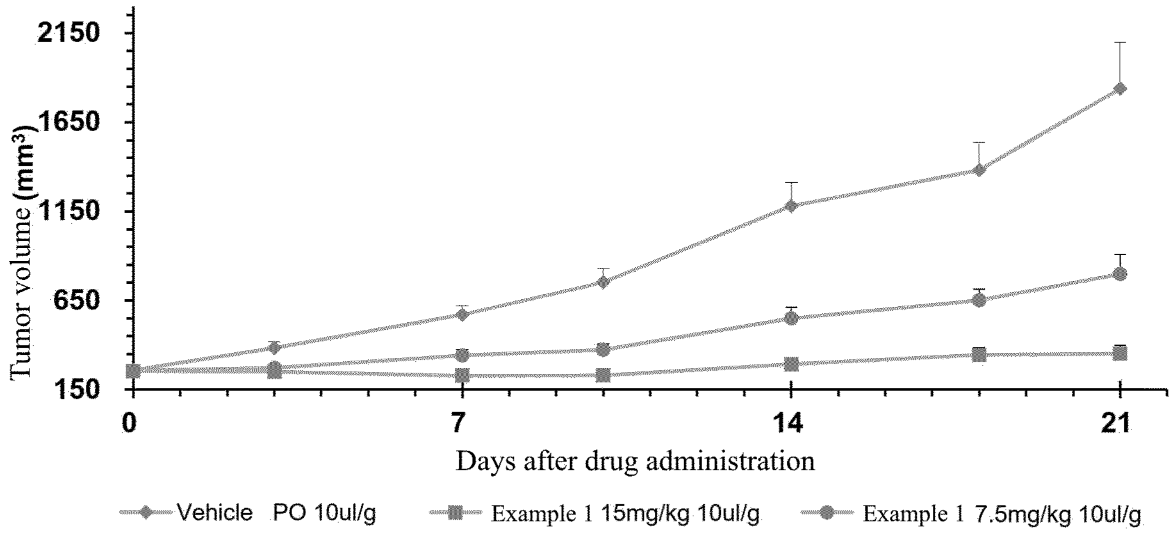

2150
1650
1150
650
150
Tumor volume (mm³)
0
7
14
21
Days after drug administration
Vehicle PO 10ul/g
Example 1 15mg/kg 10ul/g
Example 1 7.5mg/kg 10ul/g


Fig. 17

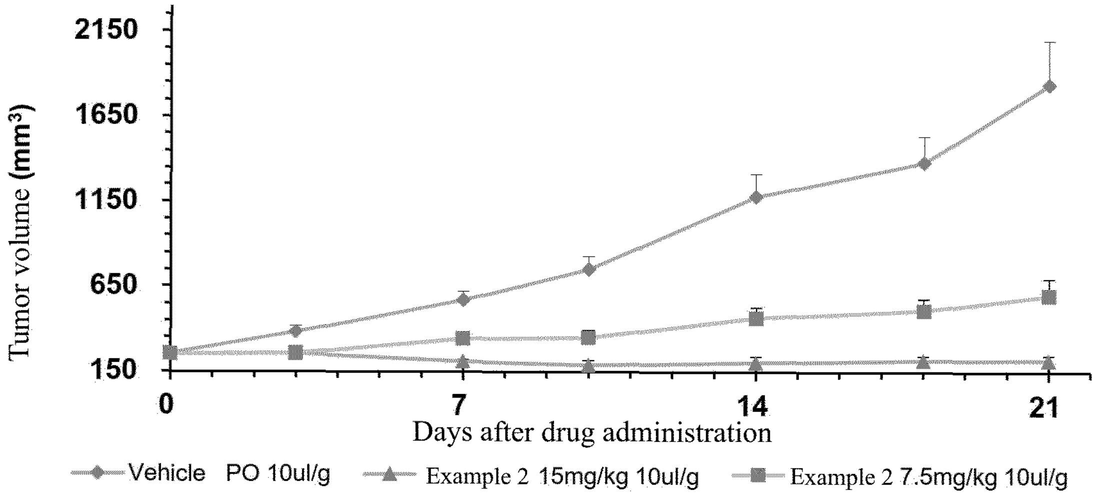
2150
1650
1150
650
150
Tumor volume (mm³)
0
7
14
21
Days after drug administration
Vehicle PO 10ul/g
Example 2 15mg/kg 10ul/g
Example 2 7.5mg/kg 10ul/g

Fig. 18

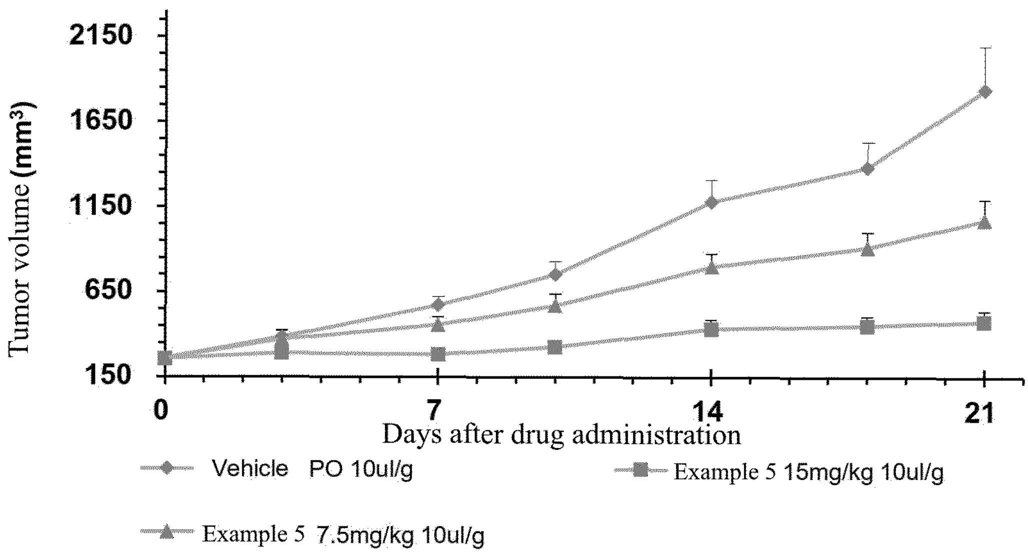
2150
1650
1150
650
150
Tumor volume (mm³)
0
7
14
21
Days after drug administration
Vehicle PO 10ul/g
Example 5 15mg/kg 10ul/g
Example 5 7.5mg/kg 10ul/g

Fig. 19

**INTERNATIONAL SEARCH REPORT**

International application No.
**PCT/CN2023/128656**

**A. CLASSIFICATION OF SUBJECT MATTER**

C07D491/056(2006.01)i; A61K31/519(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS, CNTXT, VEN, ENTXT, ENTXTC, web of science, CNKI, 万方, WANFANG, 百度, BAIDU, caplus, marpat, registry: 赛特明强, 二噁烷, 喹啉, 张强, 徐俊, 计凌涛, 盐, 对甲苯磺酸, 黏酸, 粘酸, 磷酸, 晶体, 晶型, 酪氨酸激酶, 癌, 自身免疫疾病, salt, P-toluenesulfonic acid, mucic acid, phosphoric acid, crystal?, Tyrosine kinase, VEGFR-2, C-met, c-KIT, PDGFRa, RET, AXL, NTRK, cancer?, Autoimmune, 2243235-80-3.

**C. DOCUMENTS CONSIDERED TO BE RELEVANT**

| Category* | Citation of document, with indication, where appropriate, of the relevant passages | Relevant to claim No. |
|---|---|---|
| X | WO 2019154133 A1 (BEIJING SCITECH-MQ PHARMACEUTICALS LIMITED) 15 August 2019 (2019-08-15)<br>entire document, in particular description, pages 11-13, and embodiment 5 | 1-5, 8-13 |
| A | CN 108503650 A (BEIJING SCITECH-MQ PHARMACEUTICALS LIMITED) 07 September 2018 (2018-09-07)<br>entire document | 1-13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 January 2024** | **27 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/128656**

| Patent document cited in search report | Publication date (day/month/year) | Patent family member(s) | Publication date (day/month/year) |
|---|---|---|---|
| WO 2019154133 A1 | 15 August 2019 | US 2020399285 A1 | 24 December 2020 |
| | | US 11407760 B2 | 09 August 2022 |
| | | PL 3750893 T3 | 17 July 2023 |
| | | EP 3750893 A1 | 16 December 2020 |
| | | EP 3750893 A4 | 19 April 2023 |
| | | CA 3090876 A1 | 15 August 2019 |
| | | CA 3090876 C | 06 December 2022 |
| | | JP 2021512930 A | 20 May 2021 |
| | | JP 7022454 B2 | 18 February 2022 |
| | | KR 20200118146 A | 14 October 2020 |
| | | KR 102433023 B1 | 16 August 2022 |
| | | CN 110156803 A | 23 August 2019 |
| | | CN 111788207 A | 16 October 2020 |
| CN 108503650 A | 07 September 2018 | WO 2018153293 A1 | 30 August 2018 |
| | | CN 108503650 B | 12 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019154133 A
- CN 2019073260 W


### Non-patent literature cited in the description


- **JAKEMAN et al.** *Endocrinology*, 1993, vol. 133, 848-859
- **KOLCH et al.** *Breast Cancer Research and Treatment*, 1995, vol. 36, 139-155
- **CONNOLLY et al.** *J. Biol. Chem.*, 1989, vol. 264, 20017-20024
- **KIM et al.** *Nature*, 1993, vol. 362, 841-844